# EUROPEAN PATENT APPLICATION

(11) **EP 4 664 718 A1**
(43) Date of publication of application: **17.12.2025**
(21) Application number: 24829763.2
(22) Date of filing: 22.01.2024
(51) Int. Cl.: H02J 7/00, A61B 5/145

(54) **ELECTRONIC DEVICE**

(30) Priority: 30.06.2023 CN 202310802935
(71) Applicant: Huawei Technologies Co., Ltd., Shenzhen, Guangdong 518129 (CN)
(72) Inventor: ZOU, Lin, Shenzhen, Guangdong 518129 (CN); GONG, Lexing, Shenzhen, Guangdong 518129 (CN); ZHAO, Menglong, Shenzhen, Guangdong 518129 (CN); XIE, Songlin, Shenzhen, Guangdong 518129 (CN)
(74) Representative: Maiwald GmbH
(86) International application number: PCT/CN2024/073524
(87) International publication number: WO 2025/001100

(57) **Abstract**

This application provides an electronic device, which relates to the field of power management technologies, to alleviate a problem of battery power consumption that occurs in a shelf period of the electronic device. In the electronic device, a first terminal of a first power supply is electrically connected to a first power supply end of a to-be-powered component, and a second terminal of the first power supply is electrically connected to a first end of a first metal conductive part. A cover body is detachably connected to a first assembly. When the cover body covers the first assembly, a first magnet interacts with a second magnet, so that the second magnet is electrically isolated from at least one of a second end of the first metal conductive part and a second power supply end of the to-be-powered component. When the cover body does not cover the first assembly, the second magnet is electrically connected to the first metal conductive part and the second power supply end. In a process in which the electronic device is in the shelf period or a storage period, the to-be-powered component is in an inactive state, and the power supply component does not consume power of the first power supply, so that power consumption of the first power supply can be reduced.

## Description

This application claims priority to Chinese Patent Application No. 202310802935.0, filed with the China National Intellectual Property Administration on June 30, 2023 and entitled "ELECTRONIC DEVICE", which is incorporated herein by reference in its entirety.

### TECHNICAL FIELD

This application relates to the field of power management technologies, and in particular, to an electronic device.

### BACKGROUND

With continuous development of science and technology, some functions of an electronic device need to be activated by a user in a use process after the electronic device is sold to the user. For example, when the electronic device is a continuous glucose monitoring system (continuous glucose monitoring system, CGM) device that can periodically detect a blood glucose index, in a use process, the user may first activate a transmitter of the CGM device, then attach the transmitter to the arm or abdomen of the user, and implant a blood glucose sensor under the skin, so that blood glucose values can be continuously obtained within a specific time period. A battery is usually disposed in the electronic device, and may continuously supply power to a component in the electronic device, for example, the transmitter.

However, when the electronic device is in a shelf period (stored in a warehouse or at a point of sale), the battery still needs to supply power to a detection circuit that is in the electronic device and that is configured to implement an activation function. This increases power consumption of the battery.

### SUMMARY

This application provides an electronic device, to alleviate a problem of battery power consumption that occurs in a shelf period of the electronic device.

To achieve the foregoing objective, this application uses the following technical solutions.

According to an aspect of this application, an electronic device is provided. The electronic device includes a first assembly, a cover body, and a first magnet, where the cover body is detachably connected to the first assembly. The first magnet is disposed in the cover body, and the first magnet is connected to the cover body. The first assembly includes a to-be-powered component, a first power supply, a first metal conductive part, and a second magnet. The to-be-powered component includes a first power supply end and a second power supply end. The first power supply includes a first terminal and a second terminal, and the first terminal is electrically connected to the first power supply end. A first end of the first metal conductive part is electrically connected to the second terminal. Based on this, when the cover body covers the first assembly, the first magnet interacts with the second magnet, so that the second magnet is electrically isolated from at least one of a second end of the first metal conductive part and the second power supply end. In addition, when the cover body covers the first assembly, the second magnet is electrically connected to the first metal conductive part and the second power supply end.

In conclusion, because the cover body is detachably connected to the first assembly, when a user detaches the cover body from the first assembly to make the cover body do not cover the first assembly, the first magnet connected to the cover body and the second magnet in a second assembly no longer have an effect of attracting or repelling each other, so that the second magnet can be attracted to the second end of the first metal conductive part and the second power supply end of the to-be-powered component. In this case, the second magnet is electrically connected to the first metal conductive part and the second power supply end of the to-be-powered component, so that electric energy provided by the first power supply may be transmitted to the to-be-powered component, to activate the to-be-powered component. In addition, before the to-be-powered component is activated, the cover body covers the first assembly. In this case, the first magnet connected to the cover body and the second magnet in the second assembly have the effect of attracting or repelling each other, so that the second magnet cannot be attracted to the second end of the first metal conductive part and the second power supply end of the to-be-powered component. In this case, the second magnet is electrically isolated from at least one of the second end of the first metal conductive part and the second power supply end, so that the first power supply cannot transmit electric energy to the to-be-powered component through the second magnet and the first metal conductive part. The to-be-powered component is in a non-working state, so that the to-be-powered component is in an inactive state. In this way, in comparison with a solution in which power needs to be supplied to a detection circuit in the electronic device in a shelf period or a storage period in a related technology, in a process in which the electronic device provided in embodiments of this application is in the shelf period or the storage period, the to-be-powered component is in an inactive state, and a component in the power supply component does not consume power of the first power supply, so that power consumption of the first power supply can be reduced, and a service life of the first power supply can be prolonged. In addition, when the electronic device has a small thickness and a small size, the first power supply may select a battery with a small size, for example, a button battery, to meet a design trend of miniaturization and portability of the electronic device.

In an optional implementation, when the cover body covers the first assembly, a vertical projection of the first magnet on the cover body and a vertical projection of the second magnet on the cover body overlap, and there is a preset distance between the second magnet and the first magnet. In this way, the second magnet is electrically isolated from at least the second power supply end. In this way, when the cover body can cover the first assembly, the vertical projection of the first magnet on the cover body and the vertical projection of the second magnet in the first assembly on the cover body overlap, in other words, the first magnet and the second magnet are stacked. In addition, a position of the first magnet corresponds to a position of the second magnet, so that the first magnet is located above the second magnet, and there is a preset distance between the second magnet and the first magnet. In this way, a magnetic force of the first magnet and a magnetic force of the second magnet can interact (for example, attract or repel) with each other, so that the second magnet is electrically isolated from at least the second power supply end of the to-be-powered component. In this case, the to-be-powered component is in an inactive state.

In an optional implementation, when the cover body covers the first assembly, the second magnet is electrically isolated from the second end of the first metal conductive part and the second power supply end. When the cover body does not cover the first assembly, the second magnet is attracted to the second end of the first metal conductive part. In this way, when the second magnet is attracted to at least the second end of the first metal conductive part, the second end of the first metal conductive part may be electrically connected to the second power supply end of the first power supply through the second magnet. When the second magnet is no longer attracted to the second end of the first metal conductive part, the second end of the first metal conductive part may be isolated from the second power supply endpoint of the first power supply through the second magnet.

In an optional implementation, the second magnet is disposed on a side that is of the first metal conductive part and that faces the first magnet, and magnetism of an end that is of the first magnet and that faces the second magnet is opposite to magnetism of an end that is of the second magnet and that faces the first magnet. In this way, when the cover body covers the first assembly, the first magnet may be located above the second magnet in a first direction. An attraction acting force is generated between the magnetic force of the first magnet and the magnetic force of the second magnet, so that the second magnet moves close to the first magnet in the first direction Z, to drive the second magnet to be separated from at least the first metal conductive part. Therefore, the second magnet cannot be attracted to at least the first metal conductive part. In this case, the second magnet is electrically isolated from at least the first metal conductive part, and the first power supply cannot supply power to the to-be-powered component. When the cover body does not cover the first assembly, the first magnet is no longer located above the second magnet. Therefore, the first magnet no longer attracts the second magnet. In this case, the second magnet moves in the first direction under an action of an attraction force between the first magnet and at least the first metal conductive part, and is attracted to at least the first metal conductive part. In this case, the second magnet is electrically connected to the first metal conductive part and the second power supply end of the first power supply, and the first power supply supplies power to the to-be-powered component.

In an optional implementation, the first assembly further includes an upper cover, a lower bottom, and a guide member. The lower bottom is connected to the upper cover to form a first housing. The to-be-powered component, the first power supply, the first metal conductive part, and the second magnet are located in the first housing. The lower bottom is disposed away from the first magnet relative to the upper cover. In addition, the guide member is disposed in the first housing and disposed around a periphery of the second magnet. The second magnet slidably fits the guide member in the first direction. The first direction is parallel to a direction in which the second magnet points to the first magnet. In this way, in a process in which the first magnet attracts the second magnet, the second magnet may slide in the guide member. Because the guide member is disposed around the periphery of the second magnet, a sliding direction of the second magnet may be limited through the guide member, so that in a process in which the second magnet slides in the first direction, an offset of the second magnet in a plane perpendicular to the first direction can be reduced.

In an optional implementation, an end that is of the guide member and that faces the upper cover is connected to the upper cover, there is a gap between an end that is of the guide member and that faces the lower bottom and the lower bottom, and the first metal conductive part is located in the gap. In this way, a position of at least the first metal conductive part may be limited to the gap. In the process in which the first magnet attracts the second magnet, an end that is of the first metal conductive part and that is close to the second magnet can be prevented from being tilted due to being attracted by the first magnet and the second magnet. This is not conducive to electrical isolation between the second magnet and the first metal conductive part.

In an optional implementation, the guide member includes an insulating material, and the end that is of the guide member and that faces the lower bottom abuts against the first metal conductive part. In this way, in the process in which the first magnet attracts the second magnet, because the guide member abuts against at least the first metal conductive part, an end that is of the first metal conductive part and that is close to the second magnet can be prevented from being tilted due to being attracted by the first magnet and the second magnet.

In an optional implementation, the second magnet is disposed on a side that is of the first metal conductive part and that is away from the first magnet, and magnetism of an end that is of the first magnet and that faces the second magnet is the same as magnetism of an end that is of the second magnet and that faces the first magnet. In this way, when the cover body covers the first assembly, the first magnet may be located above the second magnet in the first direction. A repulsive acting force is generated between the magnetic force of the first magnet and the magnetic force of the second magnet, so that the second magnet moves away from the first magnet in the first direction. Therefore, the second magnet cannot be attracted to the first metal conductive part. In this case, the second magnet is electrically isolated from the first metal conductive part, and the first power supply cannot supply power to the to-be-powered component. When the cover body does not cover the first assembly, the first magnet is no longer located above the second magnet. Therefore, the first magnet no longer has a repulsive acting force on the second magnet. In this case, the second magnet moves in the first direction under magnetic attraction, and is attracted to the first metal conductive part. In this case, the second magnet is electrically connected to the first metal conductive part, and the first power supply supplies power to the to-be-powered component.

In an optional implementation, the first assembly further includes an upper cover and a lower bottom. The lower bottom is connected to the upper cover to form a first housing, and the to-be-powered component, the first power supply, the first metal conductive part, and the second magnet are located in the first housing. The lower bottom is disposed away from the first magnet relative to the upper cover. A guide groove is provided on the lower bottom, and the second magnet is disposed in the guide groove. The second magnet slidably fits the guide groove in a first direction; and the first direction is parallel to a direction in which the second magnet points to the first magnet.

In an optional implementation, the first metal conductive part includes a fastening part and a cantilever that are connected to each other, and the fastening part is electrically connected to the second terminal. A part of the cantilever extends out of the first power supply. The second magnet is connected to the portion that is of the cantilever and that extends out of the first power supply. Therefore, the first metal conductive part is always electrically connected to the second magnet. In this case, when the first magnet is located above the second magnet, and the first magnet and the second magnet interact with each other (for example, attract each other), in a process in which the second magnet moves toward the first magnet in the first direction, a portion that is of the cantilever and that is connected to the second magnet may be driven to move toward the first magnet in the first direction. In this way, the second magnet may make the portion that is of the cantilever and that is connected to the second magnet be electrically isolated from the second power supply end of the first power supply. In addition, after the user opens a cover, when the first magnet is no longer located above the second magnet, and the first magnet and the second magnet do not interact with each other (for example, attract each other), the second magnet may drive, under an action of gravity in a process of moving toward the first magnet in the first direction, the portion that is of the cantilever and that is connected to the second magnet to move away from the first magnet in the first direction. In this way, the second magnet may make the portion that is of the cantilever and that is connected to the second magnet be electrically connected to the second power supply end of the to-be-powered component.

In an optional implementation, the cantilever includes a first connection part, a bent part, and a second connection part. The first connection part is connected to the fastening part, and the first connection part is disposed on a side that is of the first power supply and that faces the first magnet. The bent part is connected to the first connection part, and the bent part extends out of the first power supply. The second connection part extends out of the first power supply, the second connection part is connected to an end that is of the bent part and that is away from the first connection part, and the second connection part is further connected to the second magnet. In this way, the bent part may suspend, outside the first power supply, the second connection part connected to the second magnet, so that the second magnet drives, under an action of the magnetic force, the second connection part to move relative to the fastening part in the first direction.

In an optional implementation, in a first direction, when the cover body covers the first assembly, a distance between the second connection part and the first magnet is greater than a distance between the first connection part and the first magnet, where the first direction is parallel to a direction in which the second magnet points to the first magnet. In this way, when a second assembly is connected to the first assembly at the initial position, the distance between the second connection part and the first magnet may be greater than the distance between the first connection part and the first magnet, so that the second connection part is farther away from the first magnet than the first connection part. In this way, when the second magnet drives, under an action of the magnetic force, the second connection part to move relative to the fastening part in the first direction, sufficient moving space can be provided for the second magnet and the second connection part. This avoids a phenomenon of an erroneous electrical connection or erroneous electrical isolation between the second magnet and the second power supply end of the to-be-powered component.

In an optional implementation, the second magnet includes a magnet body and a conductive metal coating. The conductive metal coating wraps an outer surface of the magnet body. In this way, when the second magnet is attracted to at least the first metal conductive part, the conductive metal coating of the second magnet may be in direct contact with the first metal conductive part, so that a conduction capability between the second magnet and the first metal conductive part can be improved.

In an optional implementation, the first magnet includes a magnet body and a conductive metal coating. The conductive metal coating wraps an outer surface of the magnet body. Technical effects of the magnet body and the conductive metal coating are the same as those described above. Details are not described herein again.

In an optional implementation, a material of the conductive metal coating includes metal nickel. In this way, a magnet body can be protected, the magnet body is not in direct contact with an external environment, and corrosion resistance of the second magnet and the first magnet is improved.

In an optional implementation, the first assembly further includes a second power supply, where the second power supply includes a third terminal and a fourth terminal, and the fourth terminal is electrically connected to the second power supply end. The first assembly further includes a second metal conductive part, and a first end of the second metal conductive part is electrically connected to the third terminal. When the cover body covers the first assembly, the first magnet interacts with the second magnet, so that the second magnet is electrically isolated from at least one of the second end of the first metal conductive part and a second end of the second metal conductive part. When the cover body is not disposed on the first assembly, the second magnet is electrically connected to the first metal conductive part and the second metal conductive part. In this case, the first power supply and the second power supply may be connected in series. In this way, according to one aspect, in the electronic device provided in this embodiment of this application, the first magnet, the second magnet, the first metal conductive part, and the second metal conductive part may form a mechanical switch. In a process in which the electronic device is in the shelf period or the storage period, the first magnet is located above the second magnet, so that the second magnet is electrically isolated from at least one of the first metal conductive part and the second metal conductive part by using an interaction force between the first magnet and the second magnet. In this way, the to-be-powered component is in an inactive state, and a component in the power supply component do not consume power of the first power supply and the second power supply, so that a service life of the power supply is prolonged. According to another aspect, the first magnet is disposed on the cover body. When the user rotates or pulls out the cover body to open the cover body, the cover body does not cover the first assembly, and the first magnet is no longer located above the second magnet, therefore, there is no mutual acting force between the first magnet and the second magnet. In this case, the second magnet is electrically connected to the first metal conductive part and the second metal conductive part, and the to-be-powered component is in an inactive state. In this way, the to-be-powered component can be activated by when a cover is opened (that is, the cover body is opened). According to still another aspect, it can be learned from the foregoing that the first power supply, the second power supply, the first metal conductive part, the second metal conductive part, the to-be-powered component, and the like may all be located in the first housing. The cover body covers the first housing. When the user opens the cover (that is, opens the cover body), a structure of the first housing is not damaged, so that waterproof performance of each component in the first housing can be improved. Based on this, in a process in which the user wears the electronic device, a probability that external vapor enters the first housing can be reduced, thereby helping improve performance.

In an optional implementation, the first assembly further includes a second power supply. The second power supply includes a third terminal and a fourth terminal, the third terminal is electrically connected to the first power supply end, and the fourth terminal is electrically connected to the first end of the first metal conductive part. The conductive structure further includes a second metal conductive part, and a first end of the second metal conductive part is electrically connected to the second power supply end. When the cover body covers the first assembly, the first magnet interacts with the second magnet, so that the second magnet is electrically isolated from at least one of the second end of the first metal conductive part and a second end of the second metal conductive part. When the cover body is not disposed on the first assembly, the second magnet is electrically connected to the first metal conductive part and the second metal conductive part. In this case, the first power supply and the second power supply are connected in parallel. In addition, technical effects of the first magnet, the second magnet, the first power supply, the second power supply, the first metal conductive part, and the second metal conductive part are the same as those described above. Details are not described herein again.

In an optional implementation, the electronic device further includes a second housing and a bracket. The first assembly is disposed in the second housing, and the cover body and the second housing are in a threaded connection or are snap-fitted. The second housing has an opening, and when the cover body covers the first assembly, the cover body is disposed at the opening. The bracket is located in the second housing, the first assembly is located between the first magnet and the bracket, and the first assembly is disposed on the bracket. The bracket may bear the first assembly. When the cover body covers the first assembly, the cover body may cover the opening of the second housing in the first direction, so that the opening can be blocked through the cover body. This reduces external dust or external vapor entering the second housing in the shelf period or the storage period of the electronic device.

In an optional implementation, a volume of the first magnet is greater than a volume of the second magnet. In this way, in a process in which the first magnet attracts the second magnet, an attraction force of the first magnet on the second magnet may be greater than an attraction force of the first magnet on the first metal conductive part when the first magnet attracts the first metal conductive part.

In an optional implementation, the vertical projection of the first magnet on the cover body and the vertical projection of the second magnet on the cover body overlap, and the vertical projection of the second magnet on the cove body is located within a range of the vertical projection of the first magnet on the cover body. In this way, in a process in which the first magnet attracts the second magnet, an attraction force of the first magnet on the second magnet may be greater than an attraction force of the first magnet on the first metal conductive part when the first magnet attracts the first metal conductive part.

In an optional implementation, the first assembly further includes a first housing. The to-be-powered component, the first power supply, and the conductive structure are located in the first housing. In addition, the electronic device further includes a sensor. One portion of the sensor is located in the first housing, and the other portion of the sensor extends out of the first housing. The to-be-powered component includes an analog-to-digital converter, a processing chip, and a signal transmitting element. The analog-to-digital converter is electrically connected to the sensor. The processing chip is electrically connected to the sensor and the analog-to-digital converter. The signal transmitting element is electrically connected to the processing chip. In this way, the working sensor may transmit, to the analog-to-digital converter, an electrical signal generated by an oxidation reaction with glucose molecules in an ISF of the user, and the analog-to-digital converter performs analog-to-digital conversion processing on the electrical signal to generate a digital signal. The analog-to-digital converter may transmit the digital signal to the processing chip. The processing chip may further include a signal processing element that can perform data processing, for example, compression, on the digital signal, and send the digital signal to the signal transmitting element, so that the digital signal can be sent to a receiver through the signal transmitting element.

In an optional implementation, the first metal conductive part and the second metal conductive part are steel sheets, so that a magnetic attraction capability and a conduction capability between the second magnet and both the first metal conductive part and the second metal conductive part can be improved. A material of the steel sheet may include at least one of SUS430, SUS304, SUS316, SUS301, and SPCC.

### BRIEF DESCRIPTION OF DRAWINGS

FIG. 1A is a diagram of a structure of an electronic device according to an embodiment of this application;
FIG. 1B is a diagram of another structure of an electronic device according to an embodiment of this application;
FIG. 2A is a diagram of an exploded structure of an electronic device according to an embodiment of this application;
FIG. 2B is a diagram of structures of a first assembly and a sensor in FIG. 2A;
FIG. 3 is a diagram of another structure of an electronic device according to an embodiment of this application;
FIG. 4 is a diagram of a use scenario of an electronic device according to an embodiment of this application;
FIG. 5A is a diagram of a partial circuit of an electronic device according to an embodiment of this application;
FIG. 5B is a diagram of another partial circuit of an electronic device according to an embodiment of this application;
FIG. 6A is a sectional view obtained by cutting along a dashed line O1-O2 in FIG. 3;
FIG. 6B is a diagram of another partial circuit of an electronic device according to an embodiment of this application;
FIG. 7A is a diagram of a partial structure of an electronic device according to an embodiment of this application;
FIG. 7B is a diagram of a partial circuit of an electronic device corresponding to the structure shown in FIG. 7A;
FIG. 7C is a diagram of another partial circuit of an electronic device corresponding to the structure shown in FIG. 7A;
FIG. 8A is a diagram of another partial structure of an electronic device according to an embodiment of this application;
FIG. 8B is a diagram of a partial circuit of an electronic device corresponding to the structure shown in FIG. 8A;
FIG. 8C is a diagram of another partial structure of an electronic device according to an embodiment of this application;
FIG. 9 is a diagram of another partial circuit of an electronic device according to an embodiment of this application;
FIG. 10A is a diagram of another partial structure of an electronic device according to an embodiment of this application;
FIG. 10B is a sectional view obtained by cutting along a dashed line O3-O4 in FIG. 10A;
FIG. 11A is a diagram of another partial structure of an electronic device according to an embodiment of this application;
FIG. 11B is a diagram of another partial structure of an electronic device according to an embodiment of this application;
FIG. 12A is a diagram of another partial structure of an electronic device according to an embodiment of this application;
FIG. 12B is a diagram of another partial structure of an electronic device according to an embodiment of this application; and
FIG. 13 is a diagram of another partial structure of an electronic device according to an embodiment of this application.

### Reference numerals:

01: electronic device; 10: first assembly; 100: first housing; 101: to-be-powered component; 110: first power supply; 120: conductive structure; 20: second assembly; 30: sensor; 40: implant assembly; 02: bonding layer; 1011: analog-to-digital converter; 1012: processing chip; 1013: transmitting element; 200: cover body; 201: first magnet; 400: second housing; 401: bracket; 402: opening; 1202: second magnet; 1201: first metal conductive part; 151: first battery clip; 140: second power supply; 1203: second metal conductive part; 152: second battery clip; 12021: magnet body; 12022: conductive metal coating; 1001: upper cover; 1002: lower bottom; 1003: guide member; 1004: guide groove; 500: fastening part; 501: cantilever; 5011: first connection part; 5012: bent part; and 5013: second connection part.

### DESCRIPTION OF EMBODIMENTS

The following describes the technical solutions in embodiments of this application with reference to the accompanying drawings in embodiments of this application. It is clear that the described embodiments are merely a part rather than all of embodiments of this application.

The terms such as "first" and "second", below are merely for convenience of description, and are not to be construed as indicating or implying relative importance or implicitly indicating a quantity of indicated technical features. Therefore, a feature limited by "first", "second", or the like may explicitly or implicitly include one or more features. In the descriptions of this application, unless otherwise stated, "a plurality of" means two or more than two.

In this application, unless otherwise clearly specified and limited, a term "connection" should be understood in a broad sense. For example, the "connection" may be a fixed mechanical connection, or may be a detachable mechanical connection or an integrated connection, or may be a direct connection or an indirect connection implemented through an intermediate medium.

In addition, unless otherwise specified and limited, the term "electrical connection" should be understood in a broad sense. For example, the "electrical connection" may be a direct electrical connection, for example, physical contact and electrical conduction between two components, or may be understood as that different components in a circuit structure are electrically connected through a physical line that can transmit an electrical signal, for example, a printed circuit board (printed circuit board, PCB) copper foil or a wire, to transmit the electrical signal. Alternatively, the "electrical connection" may be an indirect electrical connection between two components through an intermediate medium. Alternatively, the "electrical connection" may be that two components are electrically connected in a spaced/non-contact manner, for example, the two components are electrically connected through capacitive coupling, to transmit an electrical signal. A "communication connection" may refer to an electrical signal transmission, including a wireless communication connection and a wired communication connection. The wireless communication connection does not require a physical medium and does not belong to a connection relationship that defines a construction of a product.

In embodiments of this application, "perpendicular" and "parallel" are described to respectively represent being roughly vertical and being roughly parallel within an allowed error range. The error range may be a range in which a deviation angle is less than or equal to 5°, 8°, or 10° relative to being absolutely vertical and being absolutely parallel. This is not specifically limited herein.

In embodiments of this application, orientation terms such as "upper", "lower", "left", and "right" may include but are not limited to definitions based on illustrated orientations in which components in the accompanying drawings are placed. It should be understood that these directional terms may be relative concepts, are used for description and clarification of relative positions, and may vary accordingly depending on a change in the orientations in which the components in the accompanying drawings are placed in the accompanying drawings.

In the accompanying drawings of embodiments of this application, an assembly is represented by using a guide line with an arrow, a component is represented by using only a guide line, and a hollow-out structure like an opening or a hole is represented by using a guide line with a wavy line at an end.

An embodiment of this application provides an electronic device. The electronic device may be applied to various communication systems or communication protocols, for example, a Bluetooth (Bluetooth, BT) communication technology, a global positioning system (global positioning system, GPS) communication technology, a global system for mobile communications (global system of mobile communications, GSM) communication technology, a wireless fidelity (wireless fidelity, Wi-Fi) communication technology, a wideband code division multiple access (wideband code division multiple access wireless, WCDMA) communication technology, long term evolution (long term evolution, LTE), a 5G communication technology, and another future communication technology.

The electronic device in this embodiment of this application may be a smart wearable device, for example, a medical wearable device that can detect a vital sign parameter (for example, at least one of a blood glucose index, a body temperature, a pulse, a respiratory rate, blood pressure, or blood oxygen saturation) of a user, a smart watch, a smart band, smart glasses, or a smart helmet. Alternatively, the electronic device may be a mobile phone (mobile phone), a tablet computer (pad), a notebook computer, a smart home, a virtual reality (virtual reality, VR) electronic device, an augmented reality (augmented reality, AR) electronic device, or the like. Alternatively, the electronic device may be a handheld device that has a wireless communication function, a computing device, another processing device connected to a wireless modem, a vehicle-mounted device, an electronic device in a 5G network, an electronic device in a future evolved public land mobile network (public land mobile network, PLMN), or the like. This is not limited in embodiments of this application. For ease of description, the following describes the electronic device by using an example in which the electronic device is a CGM device that can detect a blood glucose index of the user.

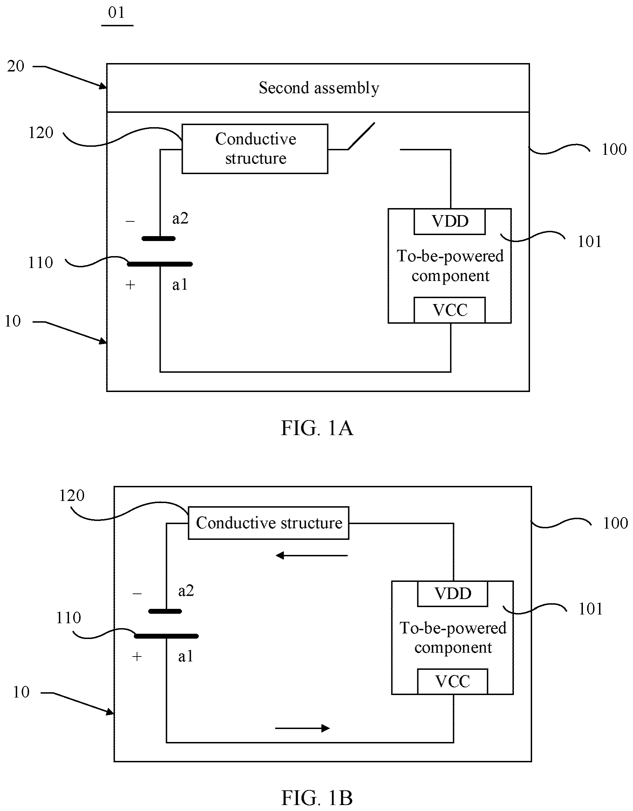

In some embodiments of this application, as shown in FIG. 1A, an electronic device 01 may include a first assembly 10 and a second assembly 20, and the first assembly 10 is detachably connected to the second assembly 20. The first assembly 10 may be a component configured to implement a main function of the electronic device 01. For example, when the electronic device 01 is the CGM device, the first assembly 10 may be a transmitter assembly that is of the CGM device and that is configured to collect the blood glucose index. When the electronic device 01 is in an unused state, the second assembly 20 may be a cover body assembly that covers the first assembly 10 (for example, the transmitter assembly). When the electronic device 01 is in a use state, the user may detach the second assembly 20 (namely, the cover body assembly) from the first assembly 10 (for example, the transmitter assembly), so that the first assembly 10 (for example, the transmitter assembly) is in a working state, to implement a main function of the electronic device 01. In the following embodiments, an example in which the electronic device 01 is the CGM device is used to describe structures of the first assembly 10 (for example, the transmitter assembly) and the second assembly 20 (namely, the cover body assembly) in detail.

It can be learned from the foregoing that the first assembly 10 is detachably connected to the second assembly 20. For example, the second assembly 20 may be directly detachably connected to the first assembly 10, or the second assembly 20 may be indirectly detachably connected to the first assembly 10 through another intermediate component. For example, the first assembly 10 is disposed on the intermediate component and is connected to the intermediate component, and the second assembly 20 is directly detachably connected to the intermediate component, so that the second assembly 20 can be indirectly detachably connected to the first assembly 10 through the intermediate component. In addition, to detachably connect the first assembly 10 to the second assembly 20, for example, the first assembly 10 and the second assembly 20 may be in a threaded connection, or the first assembly 10 and the second assembly 20 may be snap-fitted. In addition, the first assembly 10 may include a to-be-powered component 101, a first power supply 110, and a conductive structure 120. FIG. 1A is a diagram of a connection relationship between the first assembly 10, the second assembly 20, and an internal component of the first assembly 10, and does not constitute a limitation on a position relationship and structural sizes of the foregoing structures.

In some embodiments of this application, to accommodate the to-be-powered component 101, the first power supply 110, and the conductive structure 120, the first assembly 10 may further include a first housing 100. The to-be-powered component 101, the first power supply 110, and the conductive structure 120 may be disposed in the first housing 100. For example, the first housing 100 may be made of an insulating material, for example, a resin material. In addition, the first assembly 10 may further include a circuit board (not shown in the figure) located in the first housing 100, for example, a printed circuit board (printed circuit board, PCB). The to-be-powered component 101 and the first power supply 110 may be disposed on the circuit board, and are electrically connected to the circuit board.

Based on this, the to-be-powered component 101 may include a first power supply end VCC and a second power supply end VDD. For example, a positive voltage (represented by a "+" sign in FIG. 1A) may be provided to the first power supply end VCC, and a ground (GND) voltage, for example, a 0 V voltage or a negative voltage (represented by a "-" sign in FIG. 1A), may be provided to the second power supply end VDD. For another example, a positive voltage may be provided to the second power supply end VDD, and the ground voltage may be provided to the first power supply end VCC. For ease of description, the following uses an example in which the first power supply end VCC receives the positive voltage and the second power supply end VDD receives the ground voltage for description. In this case, after the first power supply end VCC and the second power supply end VDD receive corresponding voltages, power is supplied to the to-be-powered component 101, so that the to-be-powered component 101 can be activated, and the to-be-powered component 101 can be in a working state, thereby implementing a corresponding function of the to-be-powered component 101. A type of the to-be-powered component 101 is not limited in this application.

On this basis, still as shown in FIG. 1A, to supply power to the to-be-powered component 101, it can be learned from the foregoing that the first assembly 10 may further include a first power supply 110. The first power supply 110 may include a first terminal a1 and a second terminal a2, and the first terminal a1 may be electrically connected to the first power supply end VCC of the to-be-powered component 101. For example, the first power supply 110 may be a primary battery that cannot be charged, or the first power supply 110 is a secondary battery that can be charged. When a size of the electronic device 01 is small, for example, a thickness is small, the first power supply 110 may be a button battery.

In addition, the first terminal a1 may be a positive electrode of the first power supply 110, and the second terminal a2 may be a negative electrode of the first power supply 110. Alternatively, the second terminal a2 may be a positive electrode of the first power supply 110, and the first terminal a1 may be a negative electrode of the first power supply 110. For example, when the first power supply end VCC receives a positive voltage, because the first terminal a1 is electrically connected to the first power supply end VCC of the to-be-powered component 101, the first terminal a1 may be the positive electrode of the first power supply 110. For ease of description, the following embodiments are described by using an example in which the first terminal a1 is the positive electrode of the first power supply 110 and the second terminal a2 is the negative electrode of the first power supply 110. On this basis, in the first assembly 10, at least a portion of the conductive structure 120 may be electrically connected to the second terminal a2 of the first power supply 110. A manner of electrical connection between the conductive structure 120 and the second terminal a2 of the first power supply 110 is described by using an example in a subsequent embodiment with reference to a specific structure of the conductive structure 120.

It can be learned from the foregoing that the first assembly 10 may be detachably connected to the second assembly 20. In this case, there are two states between the first assembly 10 and the second assembly 20. For example, when the first assembly 10 is connected to the second assembly 20, the second assembly 20 covers the first assembly 10. After the user detaches the second assembly 20 from the first assembly 10 to make a connection relationship between the first assembly 10 and the second assembly 20 be released, the second assembly 20 does not cover the first assembly 10.

For example, when the second assembly 20 covers the first assembly 10, the second assembly 20 and the first assembly 10 may be connected to each other at an initial position. For example, when the electronic device 01 is in a shelf period or a storage period (for example, the electronic device is purchased by the user but is not unpacked, or is not used after being unpacked), a position at which the second assembly 20 is connected to the first assembly 10 is the initial position. Alternatively, for another example, that the second assembly 20 covers the first assembly 10 may be that, in a process of using the electronic device 01, the user changes relative positions between the second assembly 20 and the first assembly 10, so that the second assembly 20 covers the first assembly 10. However, in this case, the second assembly 20 and the first assembly 10 are no longer connected to each other at the initial position.

Based on this, when the second assembly 20 covers the first assembly 10, as shown in FIG. 1A, the conductive structure 120 may be electrically isolated from the second power supply end VDD. In this case, the first power supply 110 cannot transmit electric energy to the to-be-powered component 101 through the conductive structure 120, and therefore cannot supply power to the to-be-powered component 101.

When the user uses the electronic device 01, if the second assembly 20 and the first assembly 10 are connected to each other at the initial position, the relative positions between the second assembly 20 and the first assembly 10 are changed until the second assembly 20 is completely separated from the first assembly 10, so that the second assembly 20 does not cover the first assembly 10. A condition that the second assembly 20 covers the first assembly 10 is met, provided that there is a phenomenon that the conductive structure 120 can be electrically isolated from the second power supply end VDD. In addition, that the second assembly 20 does not cover the first assembly 10 may be that the connection relationship between the first assembly 10 and the second assembly 20 is released. In this case, as shown in FIG. 1B, the conductive structure 120 may be electrically connected to the second power supply end VDD. In this way, the to-be-powered component 101, the first power supply 110, and the conductive structure 120 are electrically connected to form a current loop, to transmit electric energy provided by the first power supply 110 to the to-be-powered component 101 in an arrow direction shown in FIG. 1B. In this way, power can be supplied to the to-be-powered component 101, so that the to-be-powered component 101 is activated and is in a working state.

In this embodiment of this application, "electrical connection" between two components (for example, the conductive structure 120 and the second power supply end VDD of the to-be-powered component 101) means that the two components are electrically connected, so that an electrical signal (for example, a current signal or a voltage signal) can be transmitted between the two components. In this way, the to-be-powered component 101, the first power supply 110, and the conductive structure 120 form a current loop, so that the to-be-powered component 101 is activated and is in a working state. The "electrical connection" may also be referred to as "conduction", a "connection", a "coupling connection", "coupling", or the like. In addition, "electrical isolation" between two components (for example, the conductive structure 120 and the second power supply end VDD of the to-be-powered component 101) means that the two components are not electrically connected to each other, and therefore the electrical signal cannot be transmitted between the two components. In this way, the to-be-powered component 101, the first power supply 110, and the conductive structure 120 cannot form the current loop, and consequently the to-be-powered component 101 cannot be powered on and cannot be in an active state.

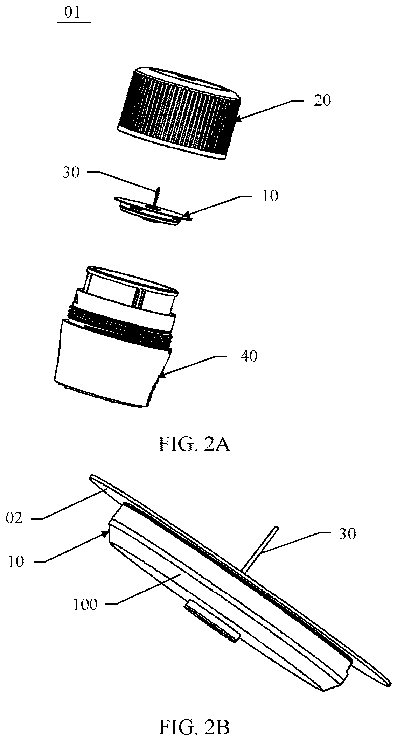

The following uses an example in which the electronic device 01 is a CGM device to describe a structure of a component in the electronic device 01 and a working principle of the electronic device 01. Based on this, as shown in FIG. 2A, the second assembly 20 of the electronic device 01 (the CGM device) may be referred to as a cover body assembly, and the first assembly 10 may be referred to as a transmitter assembly. In addition, in addition to the first assembly 10 and the second assembly 20, the electronic device 01 may further include a sensor 30 and an implant assembly 40.

In some embodiments of this application, the first assembly 10 (transmitter assembly) may include the first housing 100 shown in FIG. 2B, and the to-be-powered component 101, the first power supply 110, and the conductive structure that are 120 shown in FIG. 1B may be located in the first housing 100. In addition, still as shown in FIG. 2B, one portion of the sensor 30 may be located in the first housing 100 and electrically connected to the to-be-powered component 101, and the other portion of the sensor 30 may extend out of the first housing 100. The first assembly 10 may further include a bonding layer 02 disposed on the first housing 100, and is configured to bond the first housing 100 and the component in the first housing 100 to the skin of the user. The first assembly 10 may be disposed in the implant assembly 40.

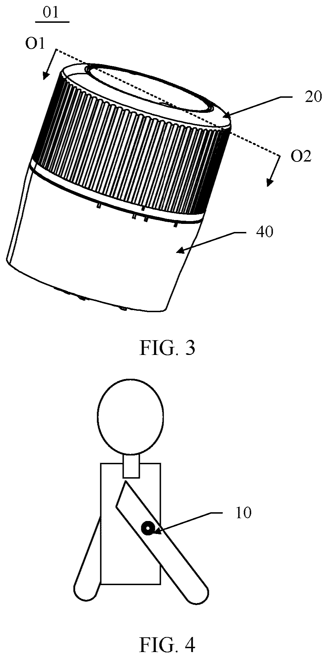

Based on this, as shown in FIG. 3, the second assembly 20 (cover body assembly) may cover the implant assembly 40, and is detachably connected to the implant assembly 40 (for example, are in a threaded connection or are snap-fitted as shown in FIG. 2A). Because the first assembly 10 shown in FIG. 2B is located in the implant assembly 40, the second assembly 20 (cover body assembly) can be indirectly detachably connected to the first assembly 10 (transmitter assembly) through the implant assembly 40.

Based on this, when the user does not use the CGM device, for example, when the CGM device is in a shelf period or a storage period, as shown in FIG. 3, the second assembly 20 may cover the implant assembly 40 and is detachably connected to the implant assembly 40. In this way, the second assembly 20 located in the implant assembly 40 covers the first assembly 10. In this case, the second assembly 20 and the first assembly 10 are connected at an initial position. For example, when the implant assembly 40 is in the threaded connection to the second assembly 20, the second assembly 20 being connected to the first assembly 10 at the initial position means that the second assembly 20 is screwed along threads on the implant assembly 40 into a position closest to the implant assembly 40. Alternatively, for another example, when the implant assembly 40 is snap-fitted on the second assembly 20, the second assembly 20 being connected to the first assembly 10 at the initial position means that the second assembly 20 is at a position on the implant assembly 40 when the second assembly 20 is snap-fitted on the implant assembly 40.

Based on this, when using the electronic device 01 (CGM device), the user may separate the second assembly 20 (cover body assembly) shown in FIG. 3 from the implant assembly 40, to expose the sensor 30 shown in FIG. 2A. Then, after the second assembly 20 is separated from the implant assembly 40, as shown in FIG. 2A, the user may push the implant assembly 40 to detach the first assembly 10 (transmitter assembly) from the implant assembly 40, and implant, under the skin of a human body, a portion that is of the sensor 30 and that is exposed to the first assembly 10 (transmitter assembly). The first assembly 10 (transmitter assembly) may be attracted to the skin (for example, an arm position) of the user, as shown in FIG. 4.

In this case, the sensor 30 implanted under the skin of the user, as shown in FIG. 2A, may perform an oxidation reaction with glucose molecules in an interstitial fluid (interstitial fluid, ISF) of the user, and generate an electrical signal. The to-be-powered component 101 in the first assembly 10 (transmitter assembly) may be used as a transmitter circuit structure having signal processing and sending functions, to perform data processing on the electrical signal from the sensor 30, and send the electrical signal to a receiver (for example, a mobile phone or a computer of the user). Because there is a high correlation between tissue fluid glucose and blood glucose that are in a human capillary, the receiver may convert, through calibration of a reference blood glucose value, an electrical signal generated by a glucose biochemical reaction in the ISF into a blood glucose detection value. In this way, the user can continuously detect a blood glucose index of the user within a period of time, to monitor a health status of the body.

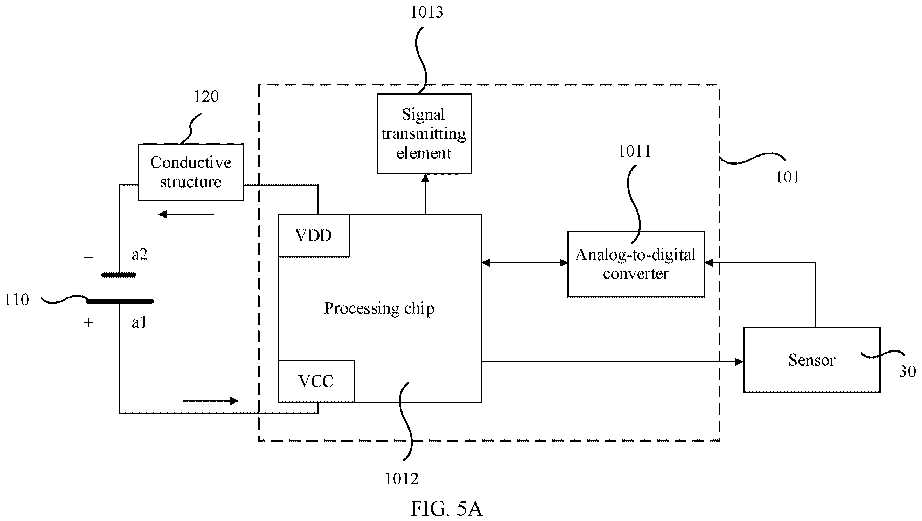

It can be learned from the foregoing that the to-be-powered component 101 can perform data processing on the electrical signal from the sensor 30 and send the electrical signal to the receiver. The to-be-powered component 101 may be referred to as a transmitter. In some embodiments of this application, as shown in FIG. 5A, the to-be-powered component 101 may include an analog-to-digital converter (analog-to-digital converter, ADC) 1011, a processing chip 1012, and a signal transmitting element 1013. The analog-to-digital converter 1011 may be electrically connected to the sensor 30. The processing chip 1012 may be electrically connected to the sensor 30 and the analog-to-digital converter 1011, and the signal transmitting element 1013 may be electrically connected to the processing chip 1012.

Based on this, in some embodiments of this application, still as shown in FIG. 5A, the first power supply end VCC and the second power supply end VDD of the to-be-powered component 101 may be disposed on the processing chip 1012. In this way, it can be learned from the foregoing that, because the second assembly 20 shown in FIG. 2A does not cover the first assembly 10, the conductive structure 120 shown in FIG. 5A may be electrically connected to the second power supply end VDD. The processing chip 1012 in the to-be-powered component 101, the first power supply 110, and the conductive structure 120 are electrically connected to form a current loop, to transmit electric energy provided by the first power supply 110 to the processing chip 1012 in an arrow direction shown in FIG. 5A. In this way, power is supplied to the processing chip 1012, to activate the entire to-be-powered component 101 (the transmitter), so that components (for example, the processing chip 1012, the analog-to-digital converter 1011, and the signal transmitting element 1013) in the to-be-powered component 101 are in a working state. For example, the processing chip 1012 may be referred to as a microprocessor or a central processing unit (central processing unit, CPU).

For example, the processing chip 1012 is electrically connected to the sensor 30. The processing chip 1012 may include a voltage conversion element, and can convert a working voltage provided by the first power supply 110, for example, a voltage of 3 V or 1.5 V (namely, a voltage difference between the first power supply end VCC and the second power supply end VDD), into a working voltage of the sensor 30 and the analog-to-digital converter 1011, to drive the sensor 30 and the analog-to-digital converter 1011 to work. The working voltage of the sensor 30 and the analog-to-digital converter 1011 may be usually less than a working voltage of the processing chip 1012.

Based on this, the working sensor 30 may transmit, to the analog-to-digital converter 1011, the electrical signal generated by the oxidation reaction with the glucose molecules in the ISF of the user, and the analog-to-digital converter 1011 performs analog-to-digital conversion processing on the electrical signal to generate a digital signal. The analog-to-digital converter 1011 may transmit the digital signal to the processing chip 1012. The processing chip 1012 may further include a signal processing element that can perform data processing, for example, compression, on the digital signal, and send the digital signal to the signal transmitting element 1013, so that the digital signal can be sent to the receiver through the signal transmitting element 1013. For example, the signal transmitting element 1013 may provide Bluetooth (Bluetooth), wireless fidelity (wireless fidelity, Wi-Fi), radio frequency (radio frequency, RF) communication, or another wireless communication manner.

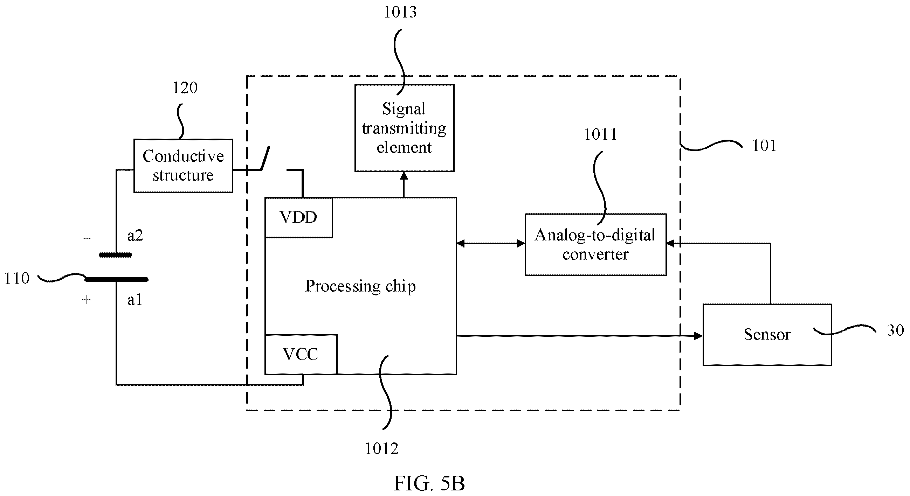

Based on this, the following describes structures of the first assembly 10 and the second assembly 20 in the electronic device 01 by using an example, so that as shown in FIG. 2A, when the second assembly 20 in the electronic device 01 does not cover the first assembly 10, the to-be-powered component 101 shown in FIG. 5A in the first assembly 10 is in an active state, and the first power supply 110 supplies power to the to-be-powered component 101. Alternatively, as shown in FIG. 3, when the second assembly 20 in the electronic device 01 covers the first assembly 10 through the implant assembly 40 (as shown in FIG. 2A), the to-be-powered component 101 shown in FIG. 5B in the first assembly 10 is in an inactive state, and the first power supply 110 does not need to supply power to the to-be-powered component 101.

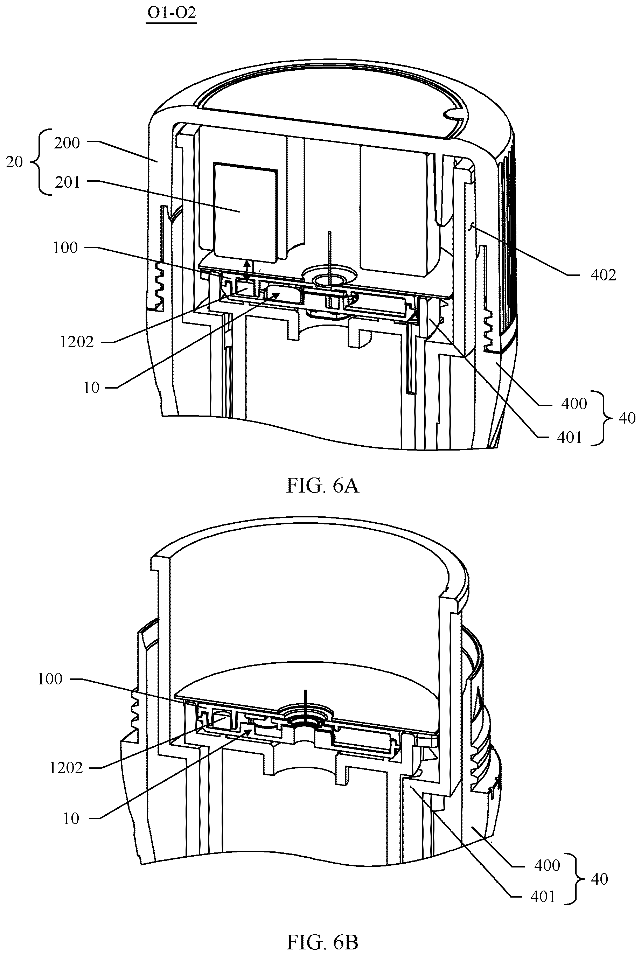

In some embodiments of this application, as shown in FIG. 6A (a sectional view obtained by cutting along O1-O2 in FIG. 3), the implant assembly 40 may include a second housing 400 and a bracket 401 located in the second housing 400. In addition, the second assembly 20 may include a cover body 200 and a first magnet 201. The second housing 400 has an opening 402. The first assembly 10 may be disposed in the second housing 400 through the opening 402, and is located on the bracket 401. The bracket 401 may carry the first assembly 10.

In addition, the cover body 200 may be detachably connected to the first assembly 10. For example, the cover body 200 in the second assembly 20 may be detachably connected to the second housing 400, for example, are in a threaded connection or are snap-fitted. FIG. 6A is described by using an example in which the cover body 200 is in a threaded connection to the second housing 400. Because the first assembly 10 is disposed in the second housing 400, the cover body 200 may be indirectly detachably connected to the first assembly 10 through the second housing 400.

Based on this, when the second assembly 20 covers the first assembly 10, the cover body 200 may cover the opening 402 of the second housing 400 in a first direction Z, so that the opening 402 can be blocked through the cover body 200. This reduces external dust or external vapor entering the second housing 400 in the shelf period or the storage period of the electronic device 01. The first direction Z may be parallel to a direction in which the first assembly 10 points to the second assembly 20, that is, the first direction Z may be a thickness direction of the electronic device 01.

Based on this, the first magnet 201 is connected to the cover body 200. For example, the first magnet 201 may be fastened to the cover body 200 through bonding, snap-fit, or the like. In this way, when the user needs to separate the second assembly 20 from the first assembly 10 to rotate or pull out the cover body 200, the cover body 200 rotates or moves relative to the implant assembly 40, and the first magnet 201 connected to the cover body 200 may move along with the cover body 200.

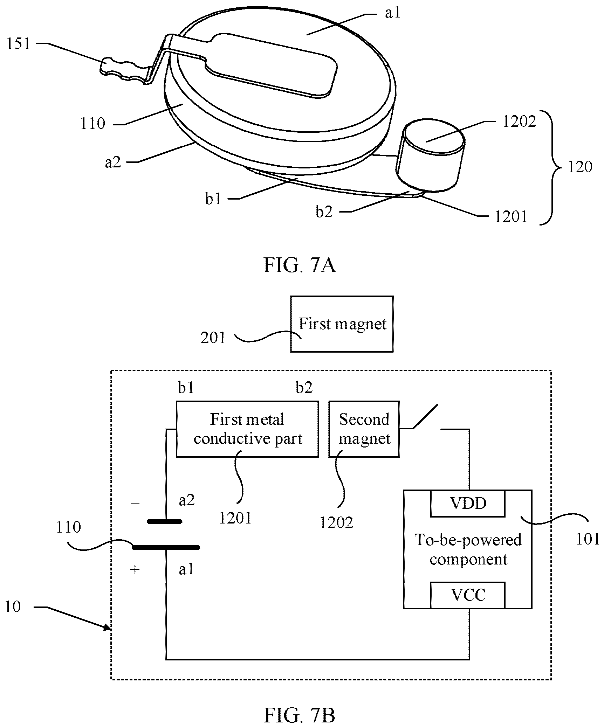

In addition, in some embodiments of this application, the conductive structure 120 in the first assembly 10 shown in FIG. 1A may include a first metal conductive part 1201 and a second magnet 1202 shown in FIG. 7A. The first metal conductive part 1201 includes a first end b1 and a second end b2 that are disposed opposite to each other. The first end b1 of the first metal conductive part 1201 may be electrically connected to the second terminal a2 of the first power supply 110. In addition, the first assembly 10 may further include a first battery clip 151. One end of the first battery clip 151 may be electrically connected to the first terminal a1 of the first power supply 110, and the other end of the first battery clip 151 may be electrically connected to the first power supply end VCC of the to-be-powered component 101 shown in FIG. 7B.

Based on this, as shown in FIG. 6A, for example, when the cover body 200 may cover the first assembly 10, the first magnet 201 and the second magnet 1202 may interact with each other, for example, magnetically attract or repel each other. In this way, the second magnet 1202 shown in FIG. 7B is electrically isolated from at least one of the second end b2 of the first metal conductive part 1201 and the second power supply end VDD of the to-be-powered component 101.

When the cover body 200 may cover the first assembly 10, the second magnet 1202 being electrically isolated from at least one of the second end b2 of the first metal conductive part 1201 and the second power supply end VDD of the to-be-powered component 101 means that, in some embodiments, the second magnet 1202 is electrically connected to the second end b2 of the first metal conductive part 1201, and the second magnet 1202 is electrically isolated from the second power supply end VDD of the to-be-powered component 101. Alternatively, in some other embodiments, the second magnet 1202 is electrically connected to the second power supply end VDD of the to-be-powered component 101, and the second magnet 1202 is electrically isolated from the second end b2 of the first metal conductive part 1201. Alternatively, in some other embodiments, the second magnet 1202 is electrically isolated from both the second end b2 of the first metal conductive part 1201 and the second power supply end VDD of the to-be-powered component 101.

Based on this, still as shown in FIG. 6A, when the cover body 200 covers the first assembly 10, a vertical projection of the first magnet 201 on the cover body 200 and a vertical projection of the second magnet 1202 in the first assembly 10 on the cover body 200 overlap, in other words, the first magnet 201 and the second magnet 1202 are stacked, and a position of the first magnet 201 corresponds to a position of the second magnet 1202, so that the first magnet 201 is located above the second magnet 1202. In addition, there may be a preset distance L between the first magnet 201 and the second magnet 1202. In this way, a magnetic force of the first magnet 201 and a magnetic force of the second magnet 1202 can interact with each other (for example, attract or repel each other), so that the second magnet 1202 shown in FIG. 7B is electrically isolated from at least the second power supply end VDD of the to-be-powered component 101.

The preset distance L between the first magnet 201 and the second magnet 1202 is not limited in this application, provided that the first magnet 201 and the second magnet 1202 generate the foregoing magnetic attraction effect or magnetic repulsion effect when a distance between the first magnet 201 and the second magnet 1202 reaches the preset distance L. In the following embodiments, a solution in which the first magnet 201 and the second magnet 1202 magnetically attract or magnetically repel each other is separately described with reference to a disposing position of the second magnet 1202.

For example, when the conductive structure 120 uses structures of the first metal conductive part 1201 and the second magnet 1202 shown in FIG. 7A, the second end b2 of the first metal conductive part 1201 and the second magnet 1202 attract each other only through magnetic forces. When the cover body 200 covers the first assembly 10, the magnetic force of the first magnet 201 interacts with the magnetic force of the second magnet 1202 in case of no other connection manner, so that the second magnet 1202 may be electrically isolated from both the second end b2 of the first metal conductive part 1201 and the second power supply end VDD of the to-be-powered component 101. For example, there is a gap between the second magnet 1202 and the second end b2 of the first metal conductive part 1201, or between the second magnet 1202 and the second power supply end VDD of the to-be-powered component 101, to ensure that the second magnet 1202 is not in direct or indirect contact with the second end b2 of the first metal conductive part 1201 and the second power supply end VDD of the to-be-powered component 101. In this case, the first power supply 110 shown in FIG. 7B cannot transmit electric energy to the to-be-powered component 101 through the first metal conductive part 1201 and the second magnet 1202, and therefore cannot supply power to the to-be-powered component 101. In this case, the to-be-powered component 101 is in an inactive state.

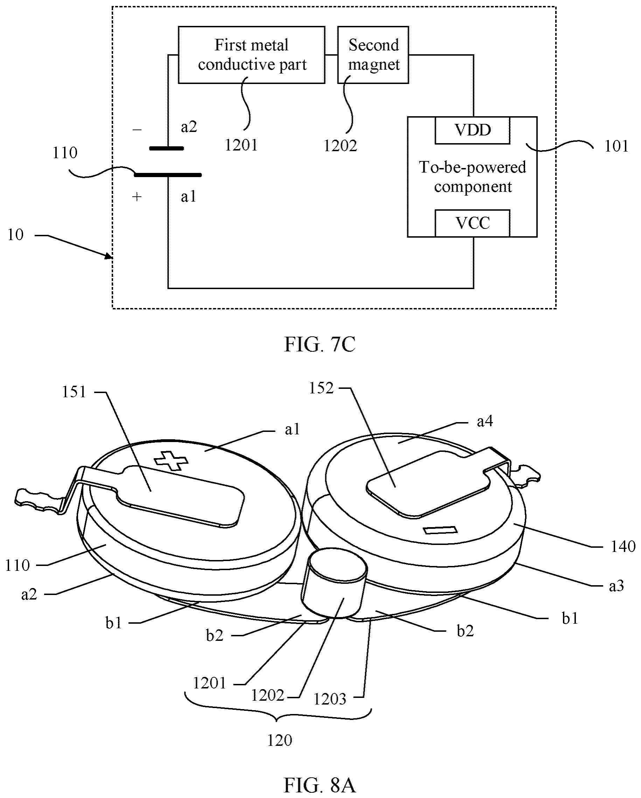

Alternatively, for another example, when the cover body 200 in the electronic device 01 does not cover the first assembly 10 shown in FIG. 6B any more, the magnetic force of the first magnet 201 on the cover body 200 no longer interacts with the magnetic force of the second magnet 1202. In this case, under a magnetic attraction effect between the second magnet 1202 and the first metal conductive part 1201, the second magnet 1202 may be attracted to the second end b2 of the first metal conductive part 1201 shown in FIG. 7A and the second power supply end VDD of the to-be-powered component 101 shown in FIG. 7C. In this way, the second magnet 1202 is electrically connected to the first metal conductive part 1201 and the second power supply end VDD of the to-be-powered component 101.

For example, when the conductive structure 120 uses structures of the first metal conductive part 1201 and the second magnet 1202 shown in FIG. 7A, the second end b2 of the first metal conductive part 1201 and the second magnet 1202 attract each other only through magnetic forces. When the magnetic force of the first magnet 201 no longer interacts with the magnetic force of the second magnet 1202 in case of no other connection manner, the second magnet 1202 may be attracted to the second end b2 of the first metal conductive part 1201, and the second power supply end VDD of the to-be-powered component 101.

In this way, when the cover body 200 in the electronic device 01 does not cover the first assembly 10, the second magnet 1202 is attracted to the second end b2 of the first metal conductive part 1201 and the second power supply end VDD of the to-be-powered component 101, so that the first power supply 110 may supply power to the to-be-powered component 101 through the first metal conductive part 1201 and the second magnet 1202. In this case, the to-be-powered component 101 is in an active state.

In conclusion, because the cover body 200 is detachably connected to the first assembly 10, when the user detaches the cover body 200 shown in FIG. 6A from the first assembly 10 (namely, transmitter assembly) to make the cover body 200 do not cover the first assembly 10 shown in FIG. 6B, the first magnet 201 connected to the cover body 200 and the second magnet 1202 in the first assembly 10 no longer have the foregoing attraction effect or repulsion effect. In this way, the second magnet 1202 may be attracted, through the magnetic attraction effect, to the second end b2 of the first metal conductive part 1201 shown in FIG. 7A and the second power supply end VDD of the to-be-powered component 101 shown in FIG. 7C. In this case, the second magnet 1202 is electrically connected to the first metal conductive part 1201 and the second power supply end VDD of the to-be-powered component 101, to supply power to the processing chip 1012. In this way, the entire to-be-powered component 101 (transmitter) is activated, so that each component in the to-be-powered component 101 is in a working state.

In addition, before the to-be-powered component 101 (transmitter) is activated, that is, when the user does not use the CGM device, as shown in FIG. 6A, the cover body 200 covers the implant assembly 40 and is detachably connected to the implant assembly 40, so that the cover body 200 may cover the first assembly 10 located in the implant assembly 40. In this case, the first magnet 201 connected to the cover body 200 and the second magnet 1202 in the first assembly 10 have the foregoing attraction effect or repulsion effect, so that the second magnet 1202 cannot be attracted to the second end b2 of the first metal conductive part 1201 shown in FIG. 7A and the second power supply end VDD of the to-be-powered component 101 shown in FIG. 7C. In this case, the second magnet 1202 may be electrically isolated from at least one of the second end b2 of the first metal conductive part 1201 and the second power supply end VDD, and the first power supply 110 cannot transmit electric energy to the to-be-powered component 101 through the conductive structure 120. The components in the to-be-powered component 101, for example, the processing chip 1012, the analog-to-digital converter 1011, and the signal transmitting element 1013 are in a non-working state, so that the to-be-powered component 101 is in an inactive state.

In this way, in comparison with a solution in which power needs to be supplied to a detection circuit in the electronic device in a shelf period or storage period in a related technology, in a process in which the electronic device 01 provided in embodiments of this application is in the shelf period or the storage period, the to-be-powered component 101 is in an inactive state, and a component in the power supply component 101 does not consume power of the first power supply 110, so that power consumption of the first power supply 110 can be reduced, and a service life of the first power supply 110 can be prolonged. In addition, when the electronic device 01 has a small thickness and a small size, the first power supply 110 may select a battery with a small size, for example, a button battery, to meet a design trend of miniaturization and portability of the electronic device 01.

The foregoing is described by using an example in which the first assembly 10 includes one power supply, namely, the first power supply 110. In some other embodiments of this application, the first assembly 10 may include two power supplies, for example, the first power supply 110 and a second power supply 140 shown in FIG. 8A. Based on this, the conductive structure 120 may further include a second metal conductive part 1203.

In addition, as shown in FIG. 8A, the second power supply 140 may include a third terminal a3 and a fourth terminal a4. The third terminal a3 may be a positive electrode of the second power supply 140, and the fourth terminal a4 may be a negative electrode of the second power supply 140. Alternatively, the third terminal a3 may be a negative electrode of the second power supply 140, and the fourth terminal a4 may be a positive electrode of the second power supply 140. For example, the first power supply 110 and the second power supply 140 may be connected in series. Based on this, when the first terminal a1 is the positive electrode of the first power supply 110 and the second terminal a2 is the negative electrode of the first power supply 110, the third terminal a3 may be the positive electrode of the second power supply 140, and the fourth terminal a4 may be the negative electrode of the second power supply 140.

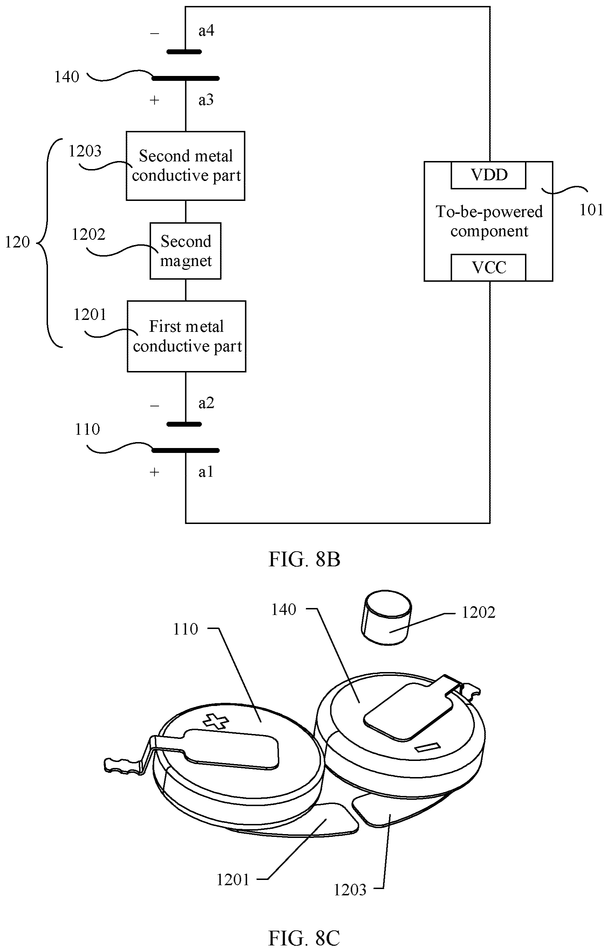

Based on this, the first assembly 10 may further include a second battery clip 152 shown in FIG. 8A. One end of the second battery clip 152 may be electrically connected to the fourth terminal a4 of the second power supply 140, and the other end of the second battery clip 152 may be electrically connected to the second power supply end VDD of the to-be-powered component 101 shown in FIG. 8B, so that the fourth terminal a4 of the second power supply 140 may be electrically connected to the second power supply end VDD of the to-be-powered component 101 through the second battery clip 152.

In addition, when the cover body 200 in the electronic device 01 does not cover the first assembly 10 shown in FIG. 6B, the magnetic force of the first magnet 201 on the cover body 200 no longer interacts with the magnetic force of the second magnet 1202. In this case, as shown in FIG. 8A, a magnetic attraction effect may be generated between the second magnet 1202 and both the first metal conductive part 1201 and the second metal conductive part 1203, so that the second magnet 1202 is attracted to the second end b2 of the second metal conductive part 1203 and the second end b2 of the first metal conductive part 1201. In this way, the second magnet 1202 may be electrically connected to the second metal conductive part 1203 and the first metal conductive part 1201.

In this way, as shown in FIG. 8B, the first power supply 110 may be connected in series to the second power supply 140 through the conductive structure 120, and may supply power to the to-be-powered component 101, to activate the to-be-powered component 101. For example, when a supply voltage of either of the first power supply 110 and the second power supply 140 is 1.5 V, after the first power supply 110 and the second power supply 140 are connected in series, a voltage of 3 V may be provided to the to-be-powered component 101.

Similarly, when the cover body 200 shown in FIG. 6A covers the first assembly 10, the first magnet 201 and the second magnet 1202 are disposed opposite to each other. Therefore, when the magnetic force of the first magnet 201 and the magnetic force of the second magnet 1202 interact with each other (for example, attract or repel each other), as shown in FIG. 8C, the second magnet 1202 is no longer attracted to the second metal conductive part 1203 and the first metal conductive part 1201. In this way, the second magnet 1202 is electrically isolated from the second metal conductive part 1203 and the first metal conductive part 1201. In this case, the first power supply 110, the second power supply 140, and the to-be-powered component 101 cannot form a current loop. Consequently, the first power supply 110 and the second power supply 140 cannot supply power to the to-be-powered component 101, and the to-be-powered component 101 is in an inactive state.

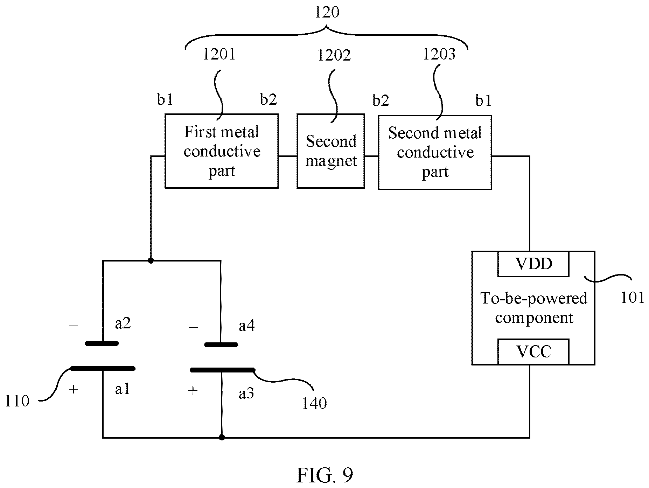

The foregoing is described by using an example in which the first power supply 110 and the second power supply 140 are connected in series and then supply power to the to-be-powered component 101. In some other embodiments of this application, the first power supply 110 and the second power supply 140 may be connected in parallel. Specifically, as shown in FIG. 9, the third terminal a3 of the second power supply 140 is electrically connected to the first power supply end VCC of the to-be-powered component 101, and the fourth terminal a4 of the second power supply 140 is electrically connected to the first end b1 of the first metal conductive part 1201. A first end b1 of the second metal conductive part 1203 is electrically connected to the second power supply end VDD of the to-be-powered component 101.

Similarly, when the cover body 200 in the electronic device 01 does not cover the first assembly 10 shown in FIG. 6B, the magnetic force of the first magnet 201 no longer interacts with the magnetic force of the second magnet 1202. In this case, as shown in FIG. 9, a magnetic attraction effect may be generated between the second magnet 1202 and both the first metal conductive part 1201 and the second metal conductive part 1203, so that the second magnet 1202 is attracted to the second end b2 of the second metal conductive part 1203 and the second end b2 of the first metal conductive part 1201. In this way, the second magnet 1202 may be electrically connected to the second metal conductive part 1203 and the first metal conductive part 1201. In this way, the first power supply 110 may be connected in parallel to the second power supply 140 through the conductive structure 120, and supply power to the to-be-powered component 101, to activate the to-be-powered component 101. For example, when a supply voltage of either of the first power supply 110 and the second power supply 140 is 1.5 V, after the first power supply 110 and the second power supply 140 are connected in series, a voltage of 1.5 V may be provided to the to-be-powered component 101.

Alternatively, when the cover body 200 shown in FIG. 6A covers the first assembly 10, the magnetic force of the first magnet 201 and the magnetic force of the second magnet 1202 interact with each other (for example, attract or repel each other), so that the second magnet 1202 is electrically isolated from at least one of the second metal conductive part 1203 and the first metal conductive part 1201. The first power supply 110 and the second power supply 140 cannot supply power to the to-be-powered component 101, and the to-be-powered component 101 is in an inactive state.

The foregoing is described by using an example in which the first assembly 10 includes two power supplies, namely, the first power supply 110 and the second power supply 140. A quantity of power supplies is not determined in embodiments of this application. When the first assembly 10 includes more than two power supplies, series and parallel connection manners of the power supplies are the same as those described above. Details are not described herein again. For ease of description, the following uses an example in which the first assembly 10 includes the first power supply 110 and the second power supply 140, and the first power supply 110 and the second power supply 140 are connected in series as shown in FIG. 8A for description.

Based on this, the following describes, by using an example, a position at which the second magnet 1202 is disposed in the first assembly 10, so that when the cover body 200 in the electronic device 01 does not cover the first assembly 10 shown in FIG. 6B, the magnetic force of the first magnet 201 no longer interacts with the magnetic force of the second magnet 1202. When the cover body 200 shown in FIG. 6A covers the first assembly 10, the magnetic force of the first magnet 201 and the magnetic force of the second magnet 1202 interact with each other (for example, attract or repel each other).

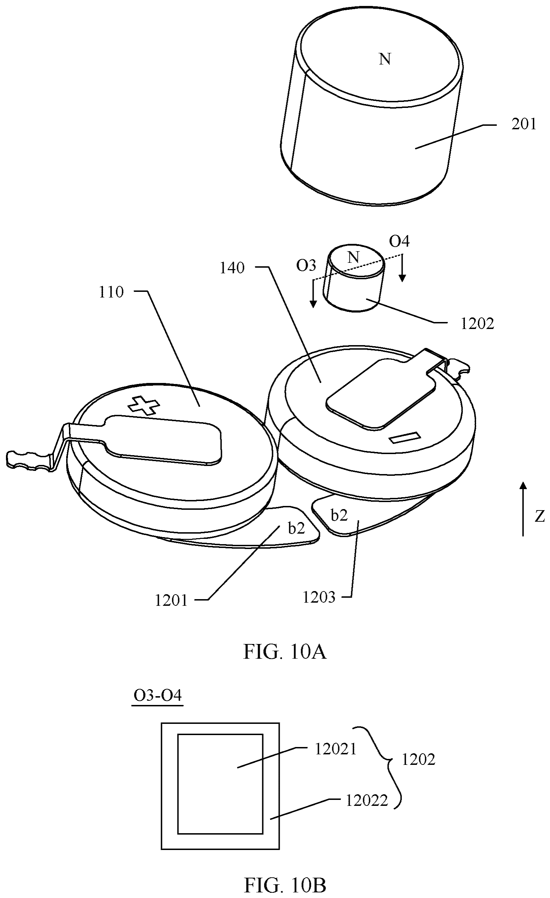

In some embodiments of this application, as shown in FIG. 10A, the second magnet 1202 is disposed on a side that is of the first metal conductive part 1201 and the second metal conductive part 1203 and that faces the first magnet 201. In this case, magnetism of an end that is of the first magnet 201 and that faces the second magnet 1202 may be opposite to magnetism of an end that is of the second magnet 1202 and that faces the first magnet 201. For example, magnetism of an upper end of the first magnet 201 is N, magnetism of a lower end (namely, the end facing the second magnet 1202) of the first magnet 201 may be S, and magnetism of an upper end (namely, the end facing the first magnet 201) of the second magnet 1202 is N. Alternatively, magnetism of an upper end of the first magnet 201 is S, magnetism of a lower end (namely, the end facing the second magnet 1202) of the first magnet 201 may be N, and magnetism of an upper end (namely, the end facing the first magnet 201) of the second magnet 1202 is S.

In this way, when the cover body 200 shown in FIG. 6A covers the first assembly 10, as shown in FIG. 10A, the first magnet 201 may be located above the second magnet 1202 in a first direction Z (namely, a direction in which the second magnet 1202 points to the first magnet 201). An attraction acting force is generated between the magnetic force of the first magnet 201 and the magnetic force of the second magnet 1202, so that the second magnet 1202 moves close to the first magnet 201 in the first direction Z, to drive the second magnet 1202 to be separated from the first metal conductive part 1201 and the second metal conductive part 1203. Therefore, the second magnet 1202 cannot be attracted to the first metal conductive part 1201 and the second metal conductive part 1203. In this case, the second magnet 1202 is electrically isolated from the first metal conductive part 1201 and the second metal conductive part 1203, and the first power supply 110 and the second power supply 140 cannot supply power to the to-be-powered component 101.

When the cover body 200 covers the first assembly 10, an example in which the second magnet 1202 shown in FIG. 10A is electrically isolated from both the second end b2 of the first metal conductive part 1201 and the second end b2 of the second metal conductive part 1203 is used for description. In this embodiment of this application, when the cover body 200 covers the first assembly 10, the second magnet 1202 may be electrically isolated from at least one of the second end b2 of the first metal conductive part 1201 and the second end b2 of the second metal conductive part 1203. For example, in some other embodiments, when the cover body 200 covers the first assembly 10, the second magnet 1202 may be electrically connected to the second end b2 of the first metal conductive part 1201, and is electrically isolated from the second end b2 of the second metal conductive part 1203. Alternatively, in some other embodiments, when the cover body 200 covers the first assembly 10, the second magnet 1202 may be electrically connected to the second end b2 of the second metal conductive part 1203, and is electrically isolated from the second end b2 of the first metal conductive part 1201.

Based on this, in a process in which the first magnet 201 attracts the second magnet 1202 shown in FIG. 10A, an attraction force of the first magnet 201 on the second magnet 1202 needs to be greater than an attraction force of the first magnet 201 on the first metal conductive part 1201 and the second metal conductive part 1203, to avoid that the second magnet 1202 cannot be driven to separate from the first metal conductive part 1201 and the second metal conductive part 1203 as the first magnet 201 cannot attract the second magnet 1202 due to a large attraction force of the second magnet 1202 on the first metal conductive part 1201 and the second metal conductive part 1203. In this case, for example, a volume of the first magnet 201 may be greater than a volume of the second magnet 1202. Alternatively, for another example, when the vertical projection of the first magnet 201 on the cover body 200 and the vertical projection of the second magnet 1202 on the cover body 200 overlap (as shown in FIG. 6A), the vertical projection of the second magnet 1202 on the cover body 200 is located within a range of the vertical projection of the first magnet 201 on the cover body 200. In this way, the magnetic force of the first magnet 201 is greater than the magnetic force of the second magnet 1202 through size setting.

In addition, when the cover body 200 in the electronic device 01 does not cover the first assembly 10 shown in FIG. 6B, the first magnet 201 shown in FIG. 10A is no longer located above the second magnet 1202, and therefore, the first magnet 201 no longer attracts the second magnet 1202. In this case, the second magnet 1202 moves in the first direction Z under an action of an attraction force of the second magnet 1202 on the first metal conductive part 1201 and the second metal conductive part 1203, and is attracted to the first metal conductive part 1201 and the second metal conductive part 1203. In this case, the second magnet 1202 is electrically connected to the first metal conductive part 1201 and the second metal conductive part 1203, and the first power supply 110 and the second power supply 140 supply power to the to-be-powered component 101.

Based on this, when the second magnet 1202 is attracted to the first metal conductive part 1201 and the second metal conductive part 1203, to improve a conducting capability between the second magnet 1202 and both the first metal conductive part 1201 and the second metal conductive part 1203, the second magnet 1202 may include a magnet body 12021 and a conductive metal coating 12022 shown in FIG. 10B (a sectional view obtained by cutting along 03-04 in FIG. 10A). The conductive metal coating 12022 may wrap an outer surface of the magnet body 12021. In this way, when the second magnet 1202 attracts the first metal conductive part 1201 and the second metal conductive part 1203, the conductive metal coating 12022 of the second magnet 1202 may be in direct contact with the first metal conductive part 1201 and the second metal conductive part 1203, and therefore, the conducting capability between the second magnet 1202 and both the first metal conductive part 1201 and the second metal conductive part 1203 can be improved.

Based on this, a material of the conductive metal coating 12022 may include metal nickel, to protect the magnet body 12021, prevent the magnet body 12021 from being in direct contact with an external environment, and improve corrosion resistance of the second magnet 1202. Similarly, the first magnet 201 may also include the magnet body and the conductive metal coating. In addition, to improve a magnetic attraction capability and a conducting capability between the second magnet 1202 and both the first metal conductive part 1201 and the second metal conductive part 1203, the first metal conductive part 1201 and the second metal conductive part 1203 may be steel sheets. A material of the steel sheet may include at least one of SUS430, SUS304, SUS316, SUS301, and SPCC.

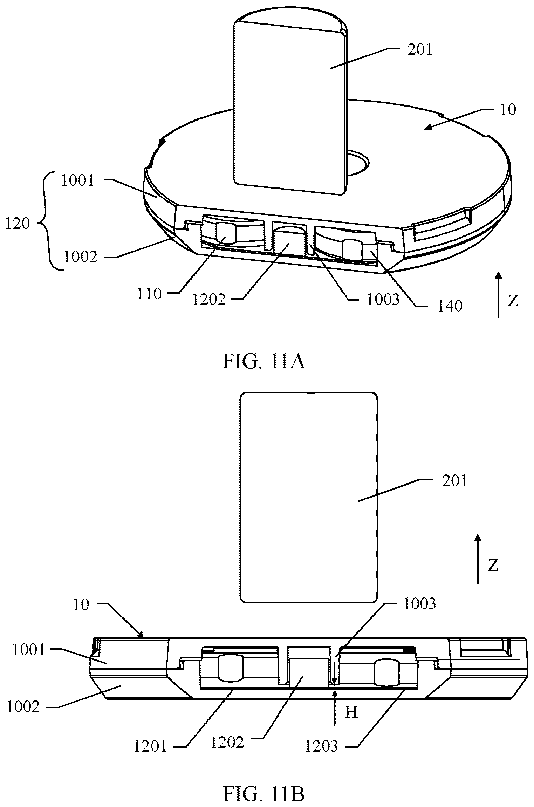

Based on this, it can be learned from the foregoing that the second magnet 1202 may move up and down in the first direction Z. In some embodiments of this application, as shown in FIG. 11A, the first assembly 10 may further include an upper cover 1001, a lower bottom 1002, and a guide member 1003, to guide the second magnet 1202 in a moving process of the second magnet 1202, and avoid that the second magnet 1202 is effectively electrically connected to or electrically isolated from the first metal conductive part 1201 and the second metal conductive part 1203 due to a shift of a moving direction of the second magnet 1202.

Specifically, still as shown in FIG. 11A, the lower bottom 1002 may be connected to the upper cover 1001. For example, the lower bottom 1002 may be connected to the upper cover 1001 through threaded connection, snap-fit, or bonding. The upper cover 1001 and the lower bottom 1002 may form the first housing 100, and the guide member 1003 is disposed in the first housing 100. The lower bottom 1002 may be disposed away from the first magnet 201 relative to the upper cover 1001. An accommodating cavity formed between the upper cover 1001 and the lower bottom 1002 may be configured to accommodate the first power supply 110, the second power supply 140, the first metal conductive part 1201, the second metal conductive part 1203, the to-be-powered component 101 (as shown in FIG. 8B), and the like shown in FIG. 10A. In addition, the guide member 1003 may be disposed around a periphery of the second magnet 1202, and the second magnet 1202 slidably fits the guide member 1003 in the first direction Z.

In this way, in a process in which the first magnet 201 attracts the second magnet 1202, the second magnet 1202 may slide in the guide member 1003. Because the guide member 1003 is disposed around the periphery of the second magnet 1202, a sliding direction of the second magnet 1202 may be limited through the guide member 1003, so that in a process in which the second magnet 1202 slides in the first direction Z, an offset of the second magnet 1202 in a plane perpendicular to the first direction Z can be reduced.

For example, as shown in FIG. 11B, an end that is of the guide member 1003 and that faces the upper cover 1001 may be connected to the upper cover. In this case, the guide member 1003 may be connected to the upper cover 1001 to form an integrated mechanical part, so that the guide member 1003 and the upper cover 1001 can be formed at the same time through a preparation process, for example, an injection molding process. In addition, still as shown in FIG. 11B, there may be a gap H between the lower bottom 1002 and an end that is of the guide member 1003 and that faces the lower bottom 1002, and the first metal conductive part 1201 and the second metal conductive part 1203 may be located in the gap H.

In this way, positions of the first metal conductive part 1201 and the second metal conductive part 1203 may be limited to the gap H. In a process in which the first magnet 201 attracts the second magnet 1202, an end that is of the first metal conductive part 1201 and the second metal conductive part 1203 and that is close to the second magnet 1202 can be prevented from being tilted due to being attracted by the first magnet 201 and the second magnet 1202. This is not conducive to electrical isolation between the second magnet 1202 and both the first metal conductive part 1201 and the second metal conductive part 1203.

Alternatively, for another example, when the guide member 1003 includes an insulating material, the end that is of the guide member 1003 and that faces the lower bottom 1002 may abut against the first metal conductive part 1201 and the second metal conductive part 1203. In this way, in a process in which the first magnet 201 attracts the second magnet 1202, the guide member 1003 abuts against the first metal conductive part 1201 and the second metal conductive part 1203. In this way, an end that is of the first metal conductive part 1201 and the second metal conductive part 1203 and that is close to the second magnet 1202 can be prevented from being tilted due to being attracted by the first magnet 201 and the second magnet 1202.

In conclusion, in one aspect, in the electronic device 01 provided in this embodiment of this application, the first magnet 201, the second magnet 1202, and the first metal conductive part 1201 or (the first metal conductive part 1201 and the second metal conductive part 1203) shown in FIG. 10A may form a mechanical switch. In a process in which the electronic device 01 is in the shelf period or the storage period, the first magnet 201 is located above the second magnet 1202, so that the second magnet 1202 is electrically isolated from the first metal conductive part 1201 or (the first metal conductive part 1201 and the second metal conductive part 1203) through an interaction acting force between the first magnet 201 and the second magnet 1202. In this way, the to-be-powered component 101 shown in FIG. 6A is in an inactive state, and a component in the power supply component 101 does not consume power of the first power supply 110 (or the first power supply 110 and the second power supply 140), so that a service life of the power supply is prolonged.

In another aspect, the first magnet 201 is disposed on the cover body 200 in FIG. 6A. When the user rotates or pulls out the cover body 200 to open the cover body 200, the first magnet 201 is no longer located above the second magnet 1202, so that there is no mutual acting force between the first magnet 201 and the second magnet 1202, the second magnet 1202 is electrically connected to the first metal conductive part 1201 or (the first metal conductive part 1201 and the second metal conductive part 1203), and the to-be-powered component 101 shown in FIG. 6A is in an inactive state. In this way, the to-be-powered component 101 can be activated when a cover is opened (that is, the cover body 200 is opened).

In still another aspect, it can be learned from the foregoing that the first power supply 110, the second power supply 140, the first metal conductive part 1201, the second metal conductive part 1203, the to-be-powered component 101, and the like may all be located in the first housing 100 shown in FIG. 6A. The cover body 200 shown in FIG. 6A covers the first housing 100. When the user opens the cover (that is, opens the cover body 200), a structure of the first housing 100 is not damaged, so that waterproof performance of each component in the first housing 100 can be improved. Based on this, in a process in which the user wears the electronic device 01, a probability that external vapor enters the first housing 100 can be reduced, thereby helping improve performance.

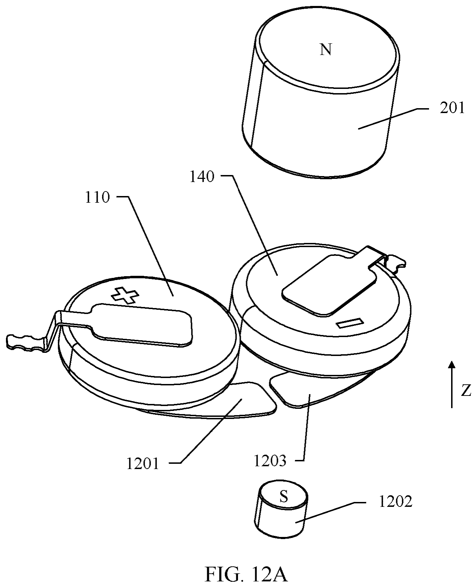

The interaction between the first magnet 201 and the second magnet 1202 is described above by using an example in which magnetism of the end that is of the first magnet 201 and that faces the second magnet 1202 may be opposite to magnetism of the end that is of the second magnet 1202 and that faces the first magnet 201. In some other embodiments of this application, as shown in FIG. 12A, the second magnet 1202 may be disposed on a side that is of the first metal conductive part 1201 and the second metal conductive part 1203 and that is away from the first magnet 201. In this case, the magnetism of the end that is of the first magnet 201 and that faces the second magnet 1202 may be the same as the magnetism of the end that is of the second magnet 1202 and that faces the first magnet 201. For example, magnetism of an upper end of the first magnet 201 is N, magnetism of a lower end (namely, the end facing the second magnet 1202) of the first magnet 201 may be S, and magnetism of an upper end (namely, the end facing the first magnet 201) of the second magnet 1202 is S. Alternatively, for another example, magnetism of an upper end of the first magnet 201 is S, magnetism of a lower end (namely, the end facing the second magnet 1202) of the first magnet 201 may be N, and magnetism of an upper end (namely, the end facing the first magnet 201) of the second magnet 1202 is N.

In this way, when the cover body 200 shown in FIG. 6A covers the first assembly 10, as shown in FIG. 12A, the first magnet 201 may be located above the second magnet 1202 in the first direction Z. A repulsive acting force is generated between the magnetic force of the first magnet 201 and the magnetic force of the second magnet 1202, so that the second magnet 1202 moves away from the first magnet 201 in the first direction Z. Therefore, the second magnet 1202 cannot be attracted to the first metal conductive part 1201 and the second metal conductive part 1203. In this case, the second magnet 1202 is electrically isolated from the first metal conductive part 1201 and the second metal conductive part 1203, and the first power supply 110 and the second power supply 140 cannot supply power to the to-be-powered component 101.

In addition, when the cover body 200 in the electronic device 01 does not cover the first assembly 10 shown in FIG. 6B, the first magnet 201 shown in FIG. 12A is no longer located above the second magnet 1202, and therefore, the first magnet 201 no longer has a repulsive acting force on the second magnet 1202. In this case, the second magnet 1202 moves in the first direction Z under magnetic attraction, and is attracted to the first metal conductive part 1201 and the second metal conductive part 1203. In this case, the second magnet 1202 is electrically connected to the first metal conductive part 1201 and the second metal conductive part 1203, and the first power supply 110 and the second power supply 140 supply power to the to-be-powered component 101.

Based on this, in a process in which the first magnet 201 and the second magnet 1202 shown in FIG. 12A repel each other, a repulsive force of the first magnet 201 on the second magnet 1202 needs to be greater than an attraction force of the second magnet 1202 on the first metal conductive part 1201 and the second metal conductive part 1203, to prevent the first magnet 201 from being attracted to the first metal conductive part 1201 and the second metal conductive part 1203. A setting manner and a technical effect of volumes and projection areas of the first magnet 201 and the second magnet 1202 are the same as those described above. Details are not described herein again.

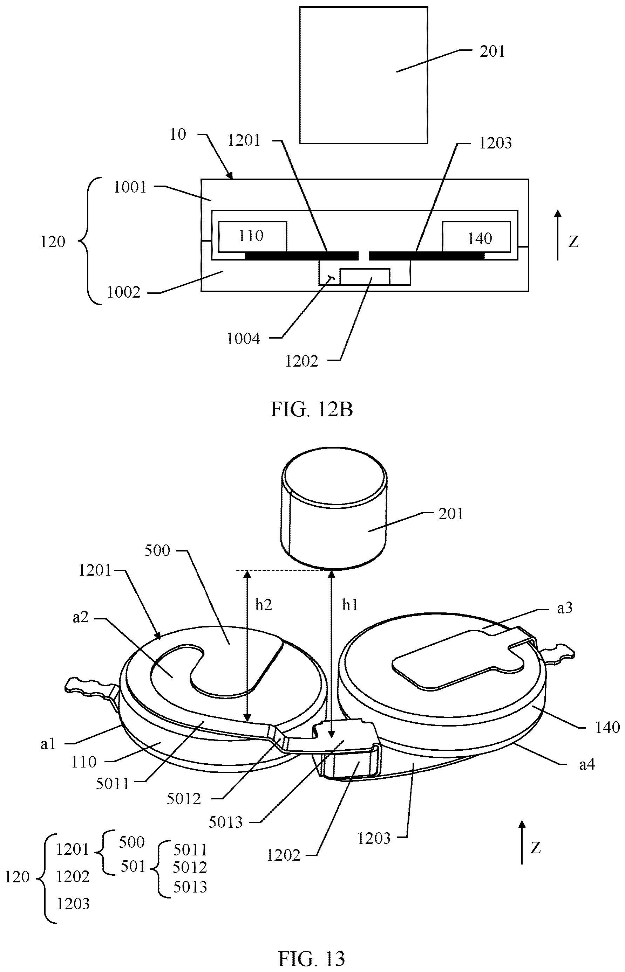

Similarly, based on this, it can be learned from the foregoing that the second magnet 1202 may move up and down in the first direction Z. To guide the second magnet 1202 in a moving process of the second magnet 1202, as shown in FIG. 12B, when the first housing 100 includes the upper cover 1001 and the lower bottom 1002 that are disposed opposite to each other and that are connected to each other, a guide groove 1004 may be provided on the lower bottom 1002. The second magnet 1202 may be disposed in the guide groove 1004, and the second magnet 1202 slidably fits the guide groove 1004, so that a sliding direction of the second magnet 1202 can be limited through the guide groove 1004. In this way, in a process in which the second magnet 1202 slides in the first direction Z, an offset of the second magnet 1202 in a plane perpendicular to the Z direction can be reduced.

When the first magnet 201 interacts with the second magnet 1202, the second magnet 1202 is electrically isolated from both the first metal conductive part 1201 and the second metal conductive part 1203. When the first magnet 201 does not interact with the second magnet 1202, an example in which the second magnet 1202 is electrically connected to both the first metal conductive part 1201 and the second metal conductive part 1203 is used for description. In some other embodiments of this application, as shown in FIG. 13, the first metal conductive part 1201 may include a fastening part 500 and a cantilever 501 that are connected to each other. The fastening part 500 is electrically connected to the second terminal a2 of the first power supply 110, and a portion of the cantilever 501 extends out of the first power supply 110. The second magnet 1202 may be connected to the portion that is of the cantilever 501 and that extends out of the first power supply 110. For example, the portion that is of the cantilever 501 and that extends out of the first power supply 110 may wrap at least a portion of the second magnet 1202. Therefore, the first metal conductive part 1201 is always electrically connected to the second magnet 1202. In addition, how the second metal conductive part 1203 is connected to the second power supply 140 is the same as that described above. Details are not described herein again.

In this case, when the first magnet 201 is located above the second magnet 1202, and the first magnet 201 and the second magnet 1202 interact with each other (for example, attract each other), in a process in which the second magnet 1202 moves toward the first magnet 201 in the first direction Z, a portion that is of the cantilever 501 and that is connected to the second magnet 1202 may be driven to move toward the first magnet 201 in the first direction Z. In this way, through the second magnet 1202, there may be a gap between the second metal conductive part 1203 and the portion that is of the cantilever 501 and that is connected to the second magnet 1202, so that the second magnet 1202 is electrically isolated from the second metal conductive part 1203. Alternatively, when the second metal conductive part 1203 and the second power supply 140 do not need to be disposed in the first assembly 10, the second magnet 1202 may make the portion that is of the cantilever 501 and that is connected to the second magnet 1202 be electrically isolated from the second power supply end VDD of the to-be-powered component 101 shown in FIG. 7B.

In addition, when the first magnet 201 is no longer located above the second magnet 1202 after the user opens the cover, and the first magnet 201 and the second magnet 1202 do not interact with each other (for example, attract each other), under the action of the attraction force between the second magnet 1202 and the second metal conductive part 1203, in a process in which the second magnet 1202 moves toward the first magnet 201 in the first direction Z, the portion that is of the cantilever 501 and that is connected to the second magnet 1202 may be driven to move away from the first magnet 201 in the first direction Z. In this way, the second magnet 1202 may make the portion that is of the cantilever 501 and that is connected to the second magnet 1202 be in contact with the second metal conductive part 1203, so that the first metal conductive part 1201 is electrically connected to the second metal conductive part 1203 through the second magnet 1202. Alternatively, when the second metal conductive part 1203 and the second power supply 140 do not need to be disposed in the first assembly 10, the second magnet 1202 may make the portion that is of the cantilever 501 and that is connected to the second magnet 1202 be electrically connected to the second power supply end VDD of the to-be-powered component 101 shown in FIG. 7C through the second magnet 1202.

The foregoing is described by using an example in which the first magnet 201 and the first metal conductive part 1201 are disposed above the second metal conductive part 1203, and the first magnet 201 and the second magnet 1202 attract each other in FIG. 13. In some other embodiments of this application, the first magnet 201 and the first metal conductive part 1201 are disposed below the second metal conductive part 1203, and when the first magnet 201 is located above the second magnet 1202, the first magnet 201 and the second magnet 1202 may repel each other. A disposing manner in which the interaction acting force between the first magnet 201 and the second magnet 1202 is an attraction acting force or a repulsive acting force is the same as that described above. Details are not described herein again.

Based on this, as shown in FIG. 13, the cantilever 501 may include a first connection part 5011, a bent part 5012, and a second connection part 5013, to drive, in the second magnet 1202, the portion that is of the cantilever 501 and that is connected to the second magnet 1202 to move toward or away from the first magnet 201 in the first direction Z. The first connection part 5011, the bent part 5012, and the second connection part 5013 are sequentially connected to each other, and the first connection part 5011, the bent part 5012, and the second connection part 5013 are electrically connected to each other.

The first connection part 5011 may be connected to the fastening part 500, and the first connection part 5011 is electrically connected to the second terminal a2 of the first power supply 110. The first connection part 5011 is disposed on a side that is of the first power supply 110 and that faces the first magnet 201. An area of the fastening part 500 may be greater than an area of the first connection part 5011, so that firmness of a connection between the entire first metal conductive part 1201 and the first power supply 110 can be ensured.

In addition, still as shown in FIG. 13, the bent part 5012 may be connected to the first connection part 5011, and the bent part 5012 extends out of the first power supply 110. The bent part 5012 may be bent in the first direction (Z direction). Based on this, the second connection part 5013 extends out of the first power supply 110, the second connection part 5013 is connected to an end that is of the bent part 5012 and that is away from the first connection part 5011, and the second connection part 5013 is further connected to the second magnet 1202. In this way, the bent part 5012 may suspend, outside the first power supply 110, the second connection part 5013 connected to the second magnet 1202, so that the second magnet 1202 drives, under an action of a magnetic force, the second connection part 5013 to move relative to the fastening part 500 in the first direction Z.

Based on this, as shown in FIG. 6A, when the cover body 200 covers the first assembly 10, a distance h1 between the second connection part 5013 shown in FIG. 13 and the first magnet 201 in the first direction Z may be greater than a distance h2 between the first connection part 5011 and the first magnet 201. In this way, when the second magnet 1202 drives, under the action of the magnetic force, the second connection part 5013 to move relative to the fastening part 500 in the first direction Z, sufficient moving space can be provided for the second magnet 1202 and the second connection part 5013 by setting h1. This avoids a phenomenon of an erroneous electrical connection or erroneous electrical isolation between the second magnet 1202 and the second metal conductive part 1203 (or the second power supply end VDD of the to-be-powered component 101 shown in FIG. 7C).

The foregoing descriptions are merely specific implementations of this application, but are not intended to limit the protection scope of this application. Any variation or replacement within the technical scope disclosed in this application shall fall within the protection scope of this application. Therefore, the protection scope of this application shall be subject to the protection scope of the claims.

## Claims

1. An electronic device, comprising:
a first assembly comprising:
a to-be-powered component, wherein the to-be-powered component comprises a first power supply end and a second power supply end;
a first power supply comprising a first terminal and a second terminal, wherein the first terminal is electrically connected to the first power supply end;
a first metal conductive part, wherein a first end of the first metal conductive part is electrically connected to the second terminal; and
a second magnet;
a cover body, detachably connected to the first assembly; and
a first magnet, disposed in the cover body and connected to the cover body, wherein
when the cover body covers the first assembly, the first magnet interacts with the second magnet, so that the second magnet is electrically isolated from at least one of a second end of the first metal conductive part and the second power supply end; and when the cover body does not cover the first assembly, the second magnet is electrically connected to the first metal conductive part and the second power supply end.

2. The electronic device according to claim 1, wherein
when the cover body covers the first assembly, a vertical projection of the first magnet on the cover body and a vertical projection of the second magnet on the cover body overlap, and there is a preset distance between the second magnet and the first magnet.

3. The electronic device according to claim 2, wherein
when the cover body covers the first assembly, the second magnet is electrically isolated from the second end of the first metal conductive part and the second power supply end; and when the cover body does not cover the first assembly, the second magnet is attracted to the second end of the first metal conductive part.

4. The electronic device according to claim 3, wherein
the second magnet is disposed on a side that is of the first metal conductive part and that faces the first magnet, and magnetism of an end that is of the first magnet and that faces the second magnet is opposite to magnetism of an end that is of the second magnet and that faces the first magnet.

5. The electronic device according to claim 4, wherein
the first assembly further comprises:
an upper cover;
a lower bottom, connected to the upper cover to form a first housing, wherein the to-be-powered component, the first power supply, the first metal conductive part, and the second magnet are located in the first housing; and the lower bottom is disposed away from the first magnet relative to the upper cover; and
a guide member, disposed in the first housing and disposed around a periphery of the second magnet, wherein the second magnet slidably fits the guide member in a first direction, and the first direction is parallel to a direction in which the second magnet points to the first magnet.

6. The electronic device according to claim 5, wherein
an end that is of the guide member and that faces the upper cover is connected to the upper cover, there is a gap between an end that is of the guide member and that faces the lower bottom and the lower bottom, and the first metal conductive part is located in the gap.

7. The electronic device according to claim 6, wherein the guide member comprises an insulating material, and the end that is of the guide member and that faces the lower bottom abuts against the first metal conductive part.

8. The electronic device according to claim 3, wherein
the second magnet is disposed on a side that is of the first metal conductive part and that is away from the first magnet, and magnetism of an end that is of the first magnet and that faces the second magnet is the same as magnetism of an end that is of the second magnet and that faces the first magnet.

9. The electronic device according to claim 8, wherein
the first assembly further comprises:
an upper cover;
a lower bottom, connected to the upper cover to form a first housing, wherein the to-be-powered component, the first power supply, the first metal conductive part, and the second magnet are located in the first housing; and the lower bottom is disposed away from the first magnet relative to the upper cover, wherein
a guide groove is provided on the lower bottom, and the second magnet is disposed in the guide groove; the second magnet slidably fits the guide groove in a first direction; and the first direction is parallel to a direction in which the second magnet points to the first magnet.

10. The electronic device according to claim 1, wherein
the first metal conductive part comprises a fastening part and a cantilever that are connected to each other, the fastening part is electrically connected to the second terminal, and a portion of the cantilever extends out of the first power supply; and
the second magnet is connected to the portion that is of the cantilever and that extends out of the first power supply.

11. The electronic device according to claim 10, wherein the cantilever comprises:
a first connection part, connected to the fastening part, wherein the first connection part is disposed on a side that is of the first power supply and that faces the first magnet;
a bent part, connected to the first connection part, wherein the bent part extends out of the first power supply; and
a second connection part extending out of the first power supply, wherein the second connection part is connected to an end that is of the bent part and that is away from the first connection part, and the second connection part is further connected to the second magnet.

12. The electronic device according to claim 11, wherein
in a first direction, when the cover body covers the first assembly, a distance between the second connection part and the first magnet is greater than a distance between the first connection part and the first magnet, wherein the first direction is parallel to a direction in which the second magnet points to the first magnet.

13. The electronic device according to any one of claims 1 to 12, wherein the second magnet comprises:
a magnet body; and
a conductive metal coating, wherein the conductive metal coating wraps an outer surface of the magnet body.

14. The electronic device according to claim 13, wherein
a material of the conductive metal coating comprises metal nickel.

15. The electronic device according to any one of claims 1 to 14, wherein
the first assembly further comprises a second power supply, wherein the second power supply comprises a third terminal and a fourth terminal, and the fourth terminal is electrically connected to the second power supply end; and
the first assembly further comprises a second metal conductive part, and a first end of the second metal conductive part is electrically connected to the third terminal, wherein
when the cover body covers the first assembly, the first magnet interacts with the second magnet, so that the second magnet is electrically isolated from at least one of the second end of the first metal conductive part and a second end of the second metal conductive part; and when the cover body does not cover the first assembly, the second magnet is electrically connected to the first metal conductive part and the second metal conductive part.

16. The electronic device according to any one of claims 1 to 14, wherein
the first assembly further comprises a second power supply, wherein the second power supply comprises a third terminal and a fourth terminal, the third terminal is electrically connected to the first power supply end, and the fourth terminal is electrically connected to the first end of the first metal conductive part; and
the first assembly further comprises a second metal conductive part, and a first end of the second metal conductive part is electrically connected to the second power supply end, wherein
when the cover body covers the first assembly, the first magnet interacts with the second magnet, so that the second magnet is electrically isolated from at least one of the second end of the first metal conductive part and a second end of the second metal conductive part; and when the cover body does not cover the first assembly, the second magnet is electrically connected to the first metal conductive part and the second metal conductive part.

17. The electronic device according to any one of claims 1 to 16, wherein the electronic device further comprises:
a second housing, wherein the first assembly is disposed in the second housing, and the cover body and the second housing are in a threaded connection or are snap-fitted; and the second housing has an opening, and when the cover body covers the first assembly, the cover body is disposed at the opening; and
a bracket, located in the second housing, wherein the first assembly is located between the first magnet and the bracket, and the first assembly is disposed on the bracket.

18. The electronic device according to any one of claims 1 to 17, wherein a volume of the first magnet is greater than a volume of the second magnet.

19. The electronic device according to any one of claims 1 to 17, wherein the vertical projection of the first magnet on the cover body and the vertical projection of the second magnet on the cover body overlap, and the vertical projection of the second magnet on the cove body is located within a range of the vertical projection of the first magnet on the cover body.

20. The electronic device according to any one of claims 1 to 19, wherein
the first assembly further comprises the first housing, wherein the to-be-powered component, the first power supply, the first metal conductive part, and the second magnet are located in the first housing;
the electronic device further comprises a sensor, wherein one portion of the sensor is located in the first housing, and the other portion of the sensor extends out of the first housing; and
the to-be-powered component comprises:
an analog-to-digital converter, electrically connected to the sensor;
a processing chip, electrically connected to the sensor and the analog-to-digital converter; and
a signal transmitting element, electrically connected to the processing chip.
